# EUROPEAN PATENT SPECIFICATION

(11) **EP 3 965 804 B1**
(45) Date of publication and mention of the grant of the patent: **02.04.2025**
(21) Application number: 20801884.6
(22) Date of filing: 05.05.2020
(51) Int. Cl.: A61K 38/46, A61K 38/50, C12N 9/14, C12N 9/86

(54) **BETA-LACTAMASE COMPOSITIONS FOR TREATMENT OF GRAFT VERSUS HOST DISEASE**
BETA-LACTAMASE-ZUSAMMENSETZUNGEN ZUR BEHANDLUNG VON GRAFT-VERSUS-HOST-REAKTION
COMPOSITIONS DE BÊTA-LACTAMASE POUR LE TRAITEMENT DE LA MALADIE DU GREFFON CONTRE L'HÔTE

(30) Priority: 06.05.2019 US 201962843849 P
(43) Date of publication of application: 16.03.2022
(73) Proprietor: Theriva Biologics, Inc., Rockville, MD 20850 (US); Memorial Sloan Kettering Cancer Center, New York, NY 10065 (US)
(72) Inventor: KALEKO, Michael, Rockville, MD 20850 (US); CONNELLY, Sheila, Rockville, MD 20850 (US); VAN DEN BRINK, Marcel R.M., New York, NY 10065 (US); PELED, Jonathan, New York, NY 10065 (US); DA SILVA, Marina Burgos, New York, NY 10065 (US)
(74) Representative: Boxall, Sarah Jane
(86) International application number: PCT/US2020/031429
(87) International publication number: WO 2020/227262

(56) References cited:
- WO-A1-2016/057744
- WO-A1-2020/030593
- WO-A1-93/13795
- US-A1- 2016 101 058
- US-A1- 2016 143 961
- US-A1- 2017 258 854
- MICHAEL KALEKO ET AL: "Development of SYN-004, an oral beta-lactamase treatment to protect the gut microbiome from antibiotic-mediated damage and prevent Clostridium difficile infection", ANAEROBE, vol. 41, 1 October 2016 (2016-10-01), AMSTERDAM, NL, pages 58 - 67, XP055444836, ISSN: 1075-9964, DOI: 10.1016/j.anaerobe.2016.05.015
- CONNELLY SHEILA ET AL: "Oral Metallo-Beta-Lactamase Protects the Gut Microbiome From Carbapenem-Mediated Damage and Reduces Propagation of Antibiotic Resistance in Pigs", FRONTIERS IN MICROBIOLOGY, vol. 10, 5 February 2019 (2019-02-05), XP093039530, DOI: 10.3389/fmicb.2019.00101
- SHONO YUSUKE ET AL: "Increased GVHD-related mortality with broad-spectrum antibiotic use after allogeneic hematopoietic stem cell transplantation in human patients and mice", SCIENCE TRANSLATIONAL MEDICINE, vol. 8, no. 339, 18 May 2016 (2016-05-18), XP093039541, ISSN: 1946-6234, DOI: 10.1126/scitranslmed.aaf2311
- FREDRICKS, DAVID N.: "The gut microbiota and graft-versus-host disease", J CLIN INVEST, vol. 129, no. 5, 1 May 2019 (2019-05-01), pages 1808 - 1817, XP055759921, DOI: 10.1172/JCI125797
- STAFFAS ET AL.: "The intestinal microbiota in allogeneic hematopoietic cell transplant and graft-versus-host disease", BLOOD, vol. 129, no. 8, 23 February 2017 (2017-02-23), pages 927 - 933, XP002787936, DOI: 10.1182/blood-2016-09-691394
- STEIN-THOERINGER ET AL.: "Domination of the Gut Microbiota with Enterococcus Species Early after Allogeneic Bone Marrow Transplantation is an Important Contributor to the Development of Acute Graft-Versus-Host Disease (GHVD", BIOL BLOOD MARROW TRANSPLANT, vol. 24, no. 3, 1 March 2018 (2018-03-01), pages S34 - S34, XP055759924
- WANG WEILIN AND SHAOYAN XU; ZHIGANG REN; JIANWEN JIANG; SHUSEN ZHENG: "Gut microbiota and allogeneic transplantation", J TRANSL MED, vol. 13, 23 August 2015 (2015-08-23), pages 1 - 11, XP021228558, DOI: 10.1186/s12967-015-0640-8
- YOSHIOKA ET AL.: "Gut microbiota and acute graft-versus-host disease", PHARMACOLOGICAL RESEARCH, vol. 122, 30 May 2017 (2017-05-30), pages 90 - 95, XP085117139, DOI: 10.1016/j.phrs.2017.05.028

## Description

### FIELD OF THE INVENTION

The present invention relates to, in part, compositions for reducing the incidence and/or severity of Graft-versus-host disease (GVHD). Particularly, the present invention provides, in part, compositions to reduce the incidence and/or severity of GVHD using one or more beta-lactamase agents.

### GOVERNMENT INTEREST

This invention was made with government support under CA008748 awarded by the National Institute of Health. The government has certain rights in the invention.

### CROSS-REFERENCE TO RELATED APPLICATIONS

This application claims the benefit of U.S. Provisional Application No. 62/843,849, filed May 6, 2019.

### DESCRIPTION OF THE TEXT FILE SUBMITTED ELECTRONICALLY

The content of the text file submitted electronically herewith is incorporated herein by reference in their entirety: A computer readable format copy of the Sequence Listing (Filename: "SYN-043PC_ST25.txt"; Date created: May 4, 2020; File size: 12,140 bytes).

### BACKGROUND

Allogenic hematopoietic stem cell transplantation (allo-HCT) can be a curative treatment for leukemia and lymphoma and is used to treat 18,000 patients annually. Graft-versus-host disease (GVHD), however, is a major complication that affects 20-50% of allo-HCT recipients. About half of GVHD cases are steroid-responsive. Steroid-refractory GVHD, and in particular gastrointestinal (Gl) GVHD, is a serious problem and accounts for about 20% of post-transplant mortality. Indeed, studies in mice have further demonstrated that treating mice having GVHD with antibiotics led to loss of the protective mucus lining of the colon and compromise of intestinal barrier function.

In particular, a profound dysbiosis of the intestinal microbiota is observed in allo-HCT patients, likely due to the broad-spectrum antibiotics that are frequently required for the empiric treatment of fever, in particular febrile neutropenia. As a result, it is thought that the intestinal microbiota in fact may modulate the risk of infection and GVHD after allo-HCT.

Currently, there are few effective treatments aimed towards mitigating GI GVHD complications. As such, novel strategies to prevent or ameliorate GVHD are needed.

### SUMMARY

In one aspect, the present invention provides a beta-lactamase for use in reducing the incidence and/or severity of graft-versus-host disease (GVHD) in a subject in need thereof, optionally wherein the GVHD is acute, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase has the amino acid sequence of SEQ ID NO: 1, SEQ ID NO: 3, SEQ ID NO: 4, or SEQ ID NO: 6, wherein the subject is being or has been administered an intravenous (IV) beta-lactam antibiotic, and wherein (i) new colonization within the intestinal microbiome of the subject is prevented, (ii) expansion of colonization within the intestinal microbiome of the subject is prevented, and/ or (iii) monodomination of the intestinal microbiome is prevented.

In another aspect, the present invention relates to a beta-lactamase for use in preventing or reducing the incidence of colonization, expansion of colonization, or monodomination and/or infection by one or more multi-drug resistant pathogens in a subject that is a transplant recipient, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase has the amino acid sequence of SEQ ID NO: 1, SEQ ID NO: 3, SEQ ID NO: 4, or SEQ ID NO: 6, and wherein the subject is being or has been administered an intravenous (IV) beta-lactam antibiotic.

In some embodiments, reducing the incidence and/or severity of complications associated with allo-HCT, such as aGVHD and/or VRE colonization and/or VRE bloodstream infection, includes preventing changes to the microbiome. For example, uses according to the present invention can result in prevention and/or attenuation of microbiome monodomination due to loss of microbial diversity and overgrowth by any given bacterial strain.

In various embodiments, a subject at risk of developing aGVHD is a transplant recipient. In further embodiments, the subject is a recipient of allo-HCT. In some embodiments, the subject is a recipient of one or more of bone marrow cells, peripheral blood cells, and umbilical cord cells.

In some embodiments, the compositions for use according to the present invention are administered prior to the transplant.

In some embodiments, the compositions for use according to the present invention are administered subsequent to the transplant.

In various embodiments, the formulations described herein act exclusively in the gastrointestinal (GI) tract and therefore provide for specific delivery while avoiding or reducing systemic exposure to beta-lactamase agents and minimizing their potential systemic effects. In various embodiments, the beta-lactamase agent is released into one or more regions of the intestinal tract. In an embodiment, the beta-lactamase agent is released into the small intestine. In another embodiment, the beta-lactamase agent is released into the large intestine.

In various embodiments, the microbiome-protecting agent (e.g., a beta-lactamase agent) may be administered to a subject in combination with additional therapeutic or preventative agents, including, but not limited to, immunosuppressive agents (e.g., cyclosporine, tacrolimus, methotrexate), antibodies, and other immune modulators that prevent aGVHD.

### BRIEF DESCRIPTION OF THE DRAWINGS

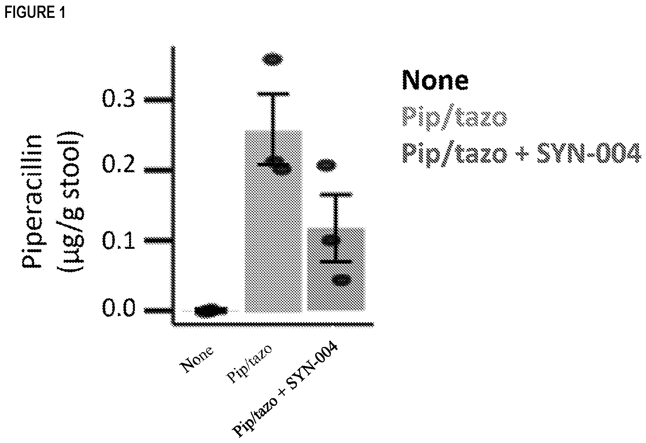

**Figure 1** shows that the amount of piperacillin (µg/g stool) was reduced in mice that received the subcutaneous administration of 500 mg/kg piperacillin/tazobactam twice per day for two days + a liquid formulation of P3A (e.g., a beta-lactamase in a phosphate-buffered saline solution) via oral gavage twice per day for two days. The leftmost bar of the bar graph corresponds to data from the control mouse cohort; the middle bar of the bar graph corresponds to data from the first cohort of mice that received piperacillin/tazobactam but did not receive P3A (e.g., a beta-lactamase in a phosphate-buffered saline solution); and the rightmost bar of the bar graph corresponds to data from the second cohort of mice that received both piperacillin/tazobactam and P3A (e.g., a beta-lactamase in a phosphate-buffered saline solution).

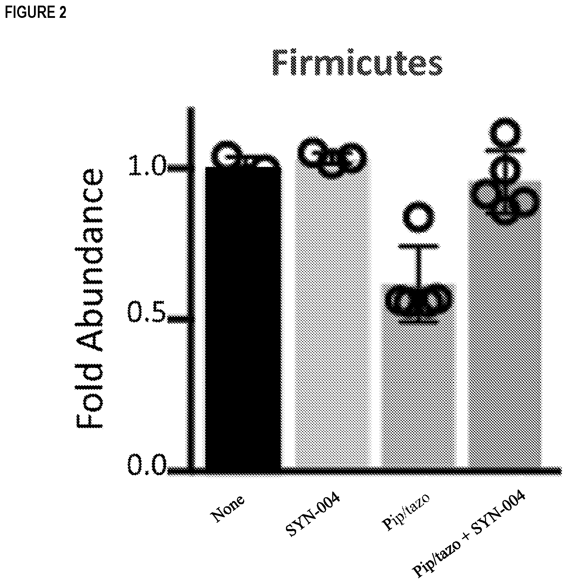

**Figure 2** shows that the abundance of *Firmicutes* in the mouse stool was reduced by piperacillin/tazobactam alone, and the fold abundance of *Firmicutes* was recovered and protected when piperacillin/tazobactam was administered with P3A. The leftmost bar of the bar graph corresponds to data from the control mouse cohort, where no piperacillin/tazobactam or beta-lactamase was administered; the bar second to the left corresponds to data from the cohort of mice that received P3A only; the bar second to the right corresponds to data from the first cohort of mice that received piperacillin/tazobactam but did not receive a liquid formulation of P3A (e.g., a beta-lactamase in a phosphate-buffered saline solution); and the rightmost bar of the bar graph corresponds to data from the second cohort of mice that received both piperacillin/tazobactam and P3A (e.g., a beta-lactamase in a phosphate-buffered saline solution).

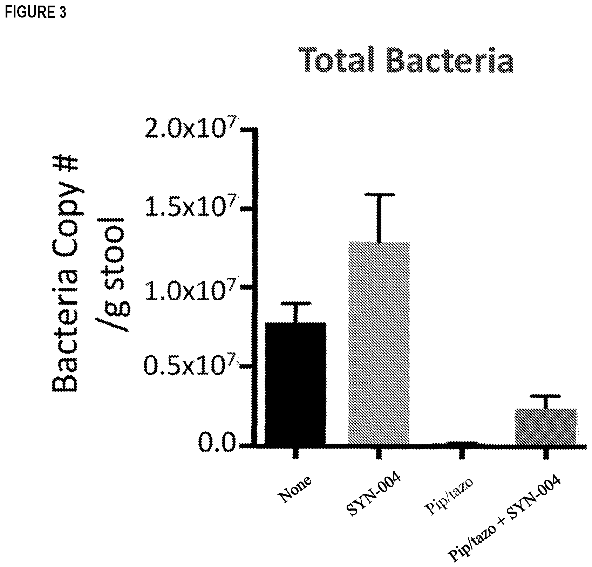

**Figure 3** shows that total bacterial copies present in the mouse stool were decreased by piperacillin/tazobactam and this decrease was reduced when piperacillin/tazobactam was administered by P3A. The leftmost bar of the bar graph corresponds to data from the control mouse cohort, where no piperacillin/tazobactam or beta-lactamase was administered; the bar second to the left corresponds to data from the cohort of mice that received P3A only; the bar second to the right corresponds to data from the first cohort of mice that received piperacillin/tazobactam but did not receive a liquid formulation of P3A (e.g., a beta-lactamase in a phosphate-buffered saline solution); and the rightmost bar of the bar graph corresponds to data from the second cohort of mice that received both piperacillin/tazobactam and P3A (e.g., a beta-lactamase in a phosphate-buffered saline solution).

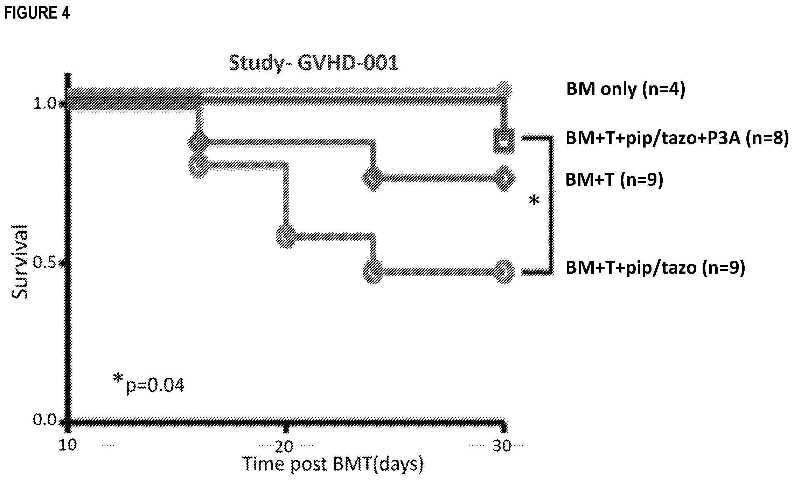

**Figure 4** shows the results of the survival study with an endpoint of 30 days post-bone marrow transplantation (BMT). The data shows that P3A administration reduced antibiotic-exacerbated GVHD associated with bone marrow transplantation in terms of reducing mortality. BM refers to a cohort that received bone marrow cells only. BM+T refers to a cohort that received bones marrow cells and splenic T lymphocyte cells.

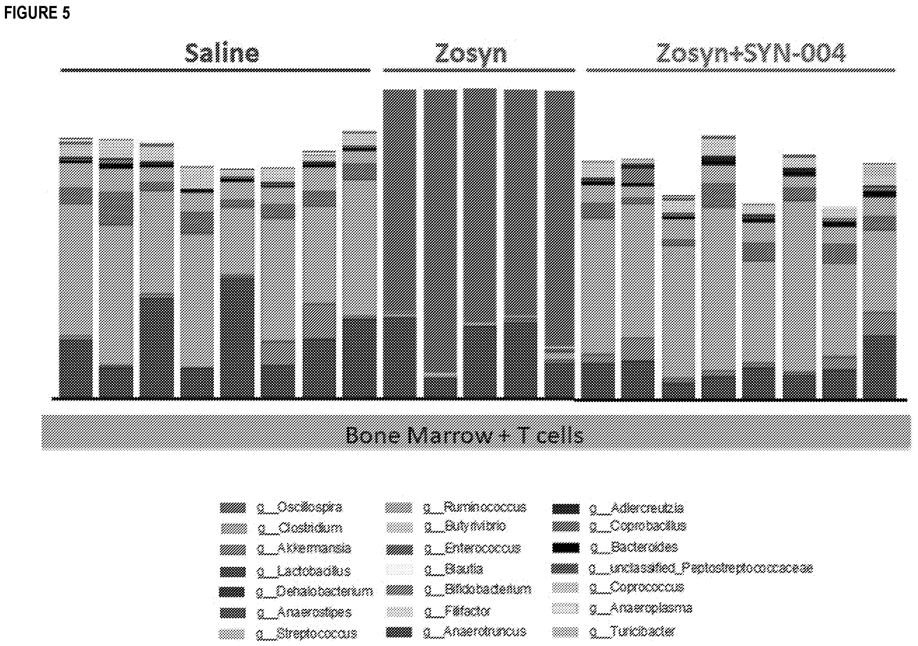

**Figure 5** depicts the results of the 16S sequencing analysis, where stool samples from mice administered both piperacillin/tazobactam (Zosyn) and SYN-004 (P3A) showed reduced *Enterococcus* monodominance compared to stool samples from mice administered piperacillin/tazobactam alone. The saline group shows the largest bars as *Clostridium;* the Zosyn group shows the largest bars as *Enterococcus;* and the Zosyn+SYN-004 group shows the largest bars as *Clostridium.*

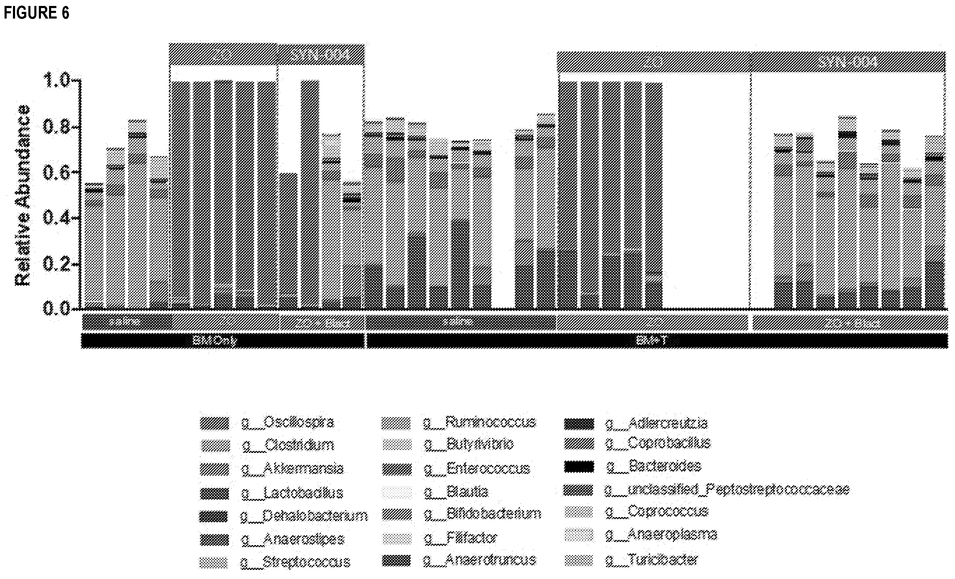

**Figure 6** depicts the results of the 16S sequencing analysis, where stool samples from mice administered both piperacillin/tazobactam (Zosyn; abbreviated as ZO) and SYN-004 (P3A; abbreviated as Blact) showed reduced *Enterococcus* monodominances compared to stool samples from mice administered ZO alone. The empty bars indicate that the particular sample did not amplify. In the BM (bone marrow) group: the saline cohort shows the largest bars as *Clostridium;* the ZO cohort shows the largest bars as *Enterococcus;* and the ZO+Blact cohort shows the largest bars as *Enterococcus* in the first two bars from the left and *Clostridium* in the next two bars from the left. In the BM (bone marrow) + T cells group: the saline cohort shows the largest bars as *Clostridium;* the ZO cohort shows the largest bars as *Enterococcus;* and the ZO+Blact cohort shows the largest bars as *Clostridium.*

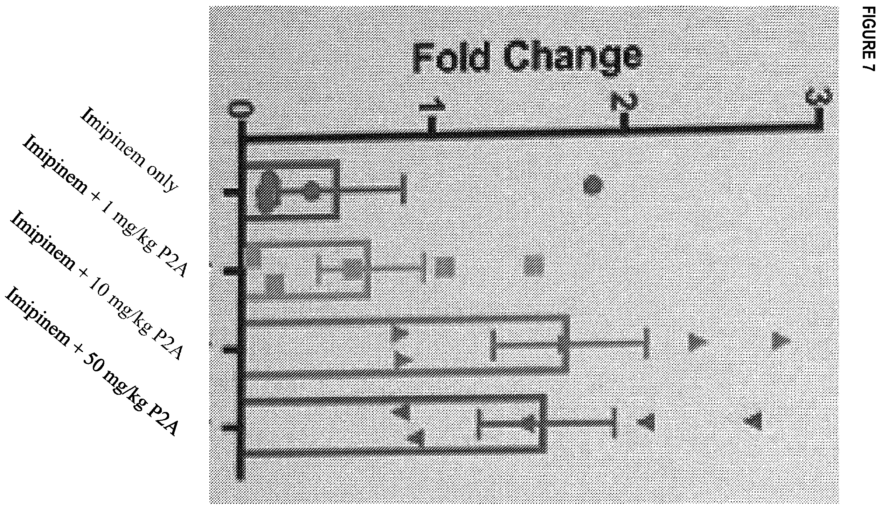

**Figure 7** shows that the fold abundance of *Firmicutes* was recovered and protected in the presence of both imipenem+cilastatin and 10 mg/kg and 50 mg/kg doses of P2A, compared to in the presence of imipenem+cilastatin alone.

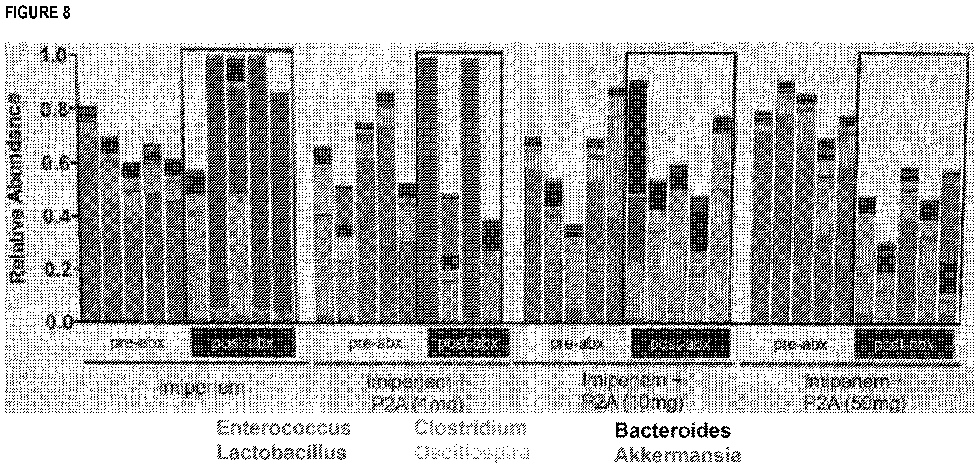

**Figure 8** depicts the results of the 16S sequencing analysis, where stool samples from mice administered both imipenem+cilastatin and SYN-006 (P2A; at 10 mg/kg and 50 mg/kg doses) showed reduced *Enterococcus* monodominance compared to stool samples from mice administered imipenem+cilastatin only. In the imipenem group, the pre-antibiotic ("pre-abx") cohort shows the largest bars as *Lactobacillus,* and the post-antibiotic ("post-abx") cohort shows the largest bars as *Enterococcus.* In the imipenem + P2A (1 mg) group, the pre-antibiotic ("pre-abx") cohort shows the largest bars as *Clostridium* and *Lactobacillus,* and the post-antibiotic ("post-abx") cohort shows the largest bars as *Enterococcus.* In the imipenem + P2A (10 mg) group, the pre-antibiotic ("pre-abx") cohort shows the largest bars as Lactobacillus, and the post-antibiotic ("post-abx") cohort shows the largest bars as *Clostridium.* In the imipenem + P2A (50 mg) group, the pre-antibiotic ("pre-abx") cohort shows the largest bars as *Lactobacillus,* and the post-antibiotic ("post-abx") cohort shows the largest bars as *Lactobacillus* and *Clostridium.*

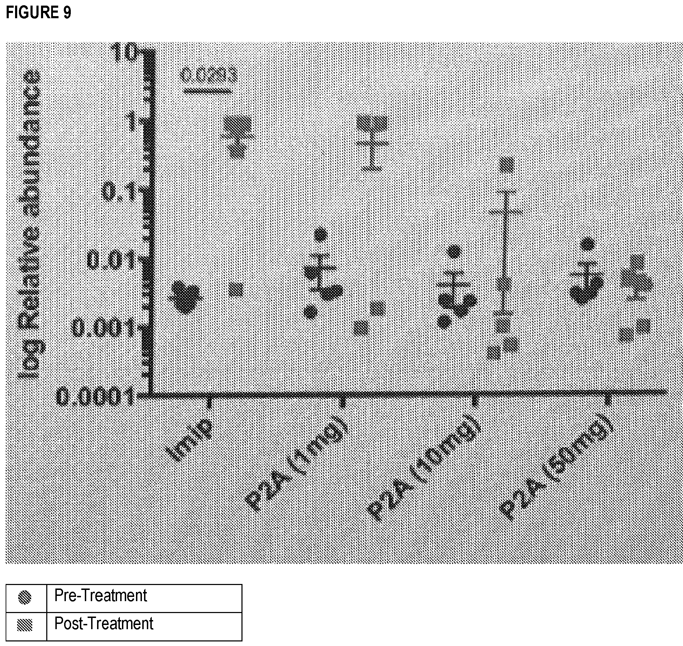

**Figure 9** shows the relative abundance of *Enterococcus faecium* in mouse stool when treated with imipenem+cilastatin alone or in combination with P2A at doses of 1 mg/kg, 10 mg/kg, and 50 mg/kg.

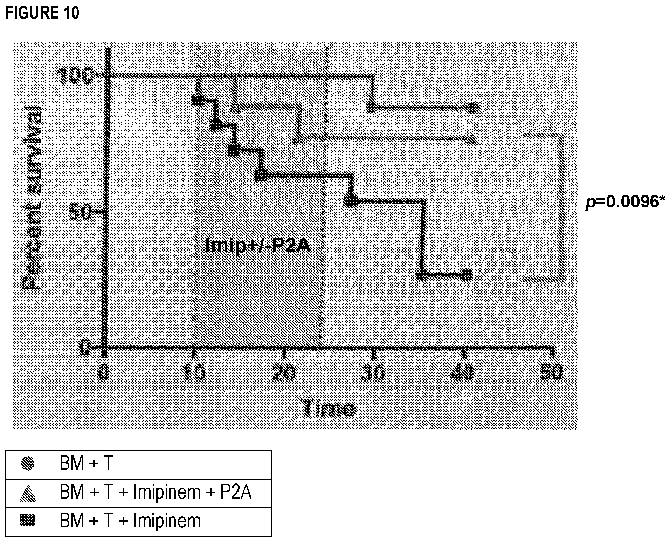

**Figure 10** shows the results of the survival study with an endpoint of 40 days post-bone marrow transplantation when administered P2A. "BM" refers to a cohort that received bone marrow cells only. "BM+T" refers to a cohort that received bones marrow cells and splenic T lymphocyte cells.

### DETAILED DESCRIPTION

In one aspect, the present invention provides a beta-lactamase for use in reducing the incidence and/or severity of graft-versus-host disease (GVHD) in a subject in need thereof, optionally wherein the GVHD is acute, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase has the amino acid sequence of SEQ ID NO: 1, SEQ ID NO: 3, SEQ ID NO: 4, or SEQ ID NO: 6, wherein the subject is being or has been administered an intravenous (IV) beta-lactam antibiotic, and wherein (i) new colonization within the intestinal microbiome of the subject is prevented, (ii) expansion of colonization within the intestinal microbiome of the subject is prevented, and/or (iii) monodomination of the intestinal microbiome is prevented.

In another aspect, the present invention relates to a beta-lactamase for use in preventing or reducing the incidence of colonization, expansion of colonization, or monodomination and/or infection by one or more multi-drug resistant pathogens in a subject that is a transplant recipient, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase has the amino acid sequence of SEQ ID NO: 1, SEQ ID NO: 3, SEQ ID NO: 4, or SEQ ID NO: 6, and wherein the subject is being or has been administered an intravenous (IV) beta-lactam antibiotic.

In various embodiments, the beta-lactamase agent is released into one or more regions of the intestinal tract where it maintains and/or modulates bacterial load and prevents the loss of bacterial strains, thereby preserving microbiome diversity and preventing microbiome monodomination.

### Beta-Lactamase Agents

In some aspects, the present invention involves uses of one or more beta-lactamases, *e.g.,* to reduce the incidence and/or severity of complications associated with IV beta-lactam antibiotic administration to allo-HCT recipients, including, but not limited to, aGVHD and VRE colonization and/or VRE bloodstream infection.

As used herein, a beta-lactamase refers to an enzyme, which hydrolyzes beta-lactams. Hydrolysis of the amide bond of the beta-lactam ring makes the antimicrobial agents biologically inactive. As used herein, class A beta-lactamases (Ambler classification) refer to serine beta-lactamases, in which the hydrolysis of the beta-lactam is mediated by serine in the active site, usually at amino acid position 70 in the alpha helix₂. Class A beta-lactamases include, but are not limited to, Len-1, SHV-1, TEM-1, PSE-3/PSE-3, ROB-1, *Bacillus cereus* such as 5/B type 1, 569/H type 1 and 569/H type 3, *Bacillus anthrasis* sp, *Bacillus licheniformis* such as PenP, *Bacillus weihenstephanensis, Bacillus clausii, Staphylococcus aureus,* PC1, Sme-1, NmcA, IMI-, PER-, VEB-, GES-, KPC-, CME- and CTX-M types beta-lactamases.

In various aspects, the beta-lactamase of use in the present invention is the amino acid sequence of SEQ ID NO: 1 (i.e., "SYN-004" or "ribaxamase" or "P3A," as described in WO 2011/14804. Mutations may be made to this sequence to generate beta-lactamase derivatives that may be utilized by the invention

SEQ ID NO: 1
TEMKDDFAKLEEQFDAKLGIFALDTGTNRTVAYRPDERFAFASTIKALTVGVL
LQQKSIEDLNQRITYTRDDLVNYNPITEKHVDTGMTLKELADASLRYSDNAAQ
NLILKQIGGPESLKKELRKIGDEVTNPERFEPELNEVNPGETQDTSTARALVT
SLRAFALEDKLPSEKRELLIDWMKRNTTGDALIRAGVPDGWEVADKTGAASY
GTRNDIAIIWPPKGDPVVLAVLSSRDKKDAKYDNKLIAEATKVVMKALNMNGK

In some embodiments, the beta-lactamase comprises an amino acid sequence having at about 95%, or about 96%, or about 97%, or about 98%, or about 99%) sequence identity with SEQ ID NO: 1.

In some embodiments, SEQ ID NO: 1 may have a Met and/or Thr preceding the first residue of the sequence. In various embodiments, the Met may be cleaved. As described herein, mutations may be made to the sequence comprising the Met and/or Thr preceding the first residue to generate beta-lactamase derivatives. In some embodiments, the leading Thr may bring about increased stability of the enzyme relative to another leading amino acid (e.g., Lys). For example, such a residue may confer increased resistance to an amino peptidase.

Also provided herein is the nucleotide sequence of SYN-004 as SEQ ID NO: 2:

SEQ ID NO: 2
atgactgagatgaaagatgattttgcgaagctggaagaacagtttgacgcaaaattgggcattttcgcgttgg
acacgggtacgaatcgtacggttgcctaccgtccggacgagcgcttcgccttcgcgagcacgatcaaagcc
ctgaccgtcggcgtgctgctccagcaaaagagcatcgaggacctgaaccagcgcattacctacacccgtg
atgatctggtgaactataatccgatcaccgagaaacacgttgataccggtatgaccctgaaagaactggca
gatgcaagcctgcgctacagcgataacgcggctcagaatctgattctgaagcaaatcggtggtccggaga
gcttgaagaaagaactgcgtaaaatcggcgatgaagtcactaatccggagcgttttgagccggagctgaac
gaagtgaatccgggtgaaacgcaagacacgagcaccgcgcgtgcgcttgtcacctccctgcgcgctttcgc
actggaagataagctgccgtcggagaaacgcgagctgctgatcgactggatgaagcgcaatacgaccgg
cgacgcgctgattcgtgcgggcgttccggacggttgggaagtggctgacaagaccggtgcggcgagctac
ggcacccgtaacgatatcgcgatcatttggccacctaaaggtgacccggtcgtgctggccgtactgagcag
ccgtgacaagaaagacgcaaagtatgataacaagctgattgcagaggcgaccaaagttgttatgaaggc
actgaacatgaatggtaag

In some embodiments, a polynucleotide of the invention may have at least about 60% (*e.g*., about 60%, or about 61%, or about 62%, or about 63%, or about 64%, or about 65%, or about 66%, or about 67%, or about 68%, or about 69%, or about 70%, or about 71%, or about 72%, or about 73%, or about 74%, or about 75%, or about 76%, or about 77%, or about 78%, or about 79%, or about 80%, or about 81%, or about 82%, or about 83%, or about 84%, or about 85%, or about 86%, or about 87%, or about 88%, or about 89%, or about 90%, or about 91%, or about 92%, or about 93%, or about 94%, or about 95%, or about 96%, or about 97%, or about 98%, or about 99%) sequence identity with SEQ ID NO: 2.

In some embodiments, the beta-lactamase, *e.g.,* SYN-004, has substantial ceftriaxone hydrolyzing activity. In some embodiments, the beta-lactamase, *e.g.,* SYN-004, hydrolyzes ceftriaxone substantially more efficiently than the naturally occurring beta-lactamase P1A.

Disclosed are the beta-lactamases comprise an amino acid sequence having at least 60% sequence identity with SEQ ID NO: 1 and the following of Ambler classification: a hydrophobic residue other than alanine (A) at position 232; a hydrophilic residue other than alanine (A) at position 237; a hydrophobic residue other than alanine (A) at position 238; a hydrophilic residue other than serine (S) at position 240; and a hydrophilic residue other than aspartate (D) at position 276. In some embodiments, the hydrophobic residue other than alanine (A) at position 232 is glycine (G). In some embodiments, the hydrophilic residue other than alanine (A) at position 237 is serine (S). In some embodiments, the hydrophobic residue other than alanine (A) at position 238 is glycine (G). In some embodiments, the hydrophilic residue other than serine (S) at position 240 is aspartate (D). In some embodiments, the other than aspartate (D) at position 276 is asparagine (N). In some embodiments, the beta-lactamase comprises one or more of A232G, A237S, A238G, S240D, and D276N.

In some aspect of the present invention, the beta-lactamase comprises all of A232G, A237S, A238G, S240D, and D276N, the sequence of which is SEQ ID NO: 3, i.e. P4A. In some embodiments, the beta-lactamase and/or pharmaceutical composition comprises an amino acid sequence having at least 95%, or 97%, or 99%, or 100% sequence identity with SEQ ID NO: 3.

SEQ ID NO: 3
EMKDDFAKLEEQFDAKLGIFALDTGTNRTVAYRPDERFAFASTIKALT
VGVLLQQKSIEDLNQRITTRDDLVNYNPITEKHVDTGMTLKELADASL
RYSDNAAQNLILKQIGGPESLKKELRKIGDEVTNPERFEPELNEVNP
GETQDTSTARALVTSLRAFALEDKLPSEKRELLIDWMKRNTTGDALI
RAGVPDGWEVGDKTGSGDYGTRNDIAIIWPPKGDPVVLAVLSSRDK
KDAKYDNKLIAEATKVVMKALNMNGK

In some aspects of the invention, the beta-lactamase polypeptide of the invention comprises an amino acid sequence having at least about 95%, or about 96%, or about 97%, or about 98%, or about 99% sequence identity with SEQ ID NO: 3.

SEQ ID NO: 4 is derived from SEQ ID NO: 3 and further includes the signal and the addition of the QASKT amino acids (the coding region is underlined):

MIQKRKRTVSFRLVLMCTLLFVSLPITKTSAQASKTEMKDDFAKLEEQFD
AKLGIFALDTGTNRTVAYRPDERFAFASTIKALTVGVLLQQKSIEDLNQRI
TYTRDDLVNYNPITEKHVDTGMTLKELADASLRYSDNAAQNLILKQIGGP
ESLKKELRKIGDEVTNPERFEPELNEVNPGETQDTSTARALVTSLRAFAL

EDKLPSEKRELLIDWMKRNTTGDALIRAGVPDGWEVGDKTGSGDYGTR
NDIAIIWPPKGDPVVLAVL SSRDKKDAKYDNKLIAEATKVVMKALNMNGK

In some aspects of the invention, the beta-lactamase polypeptide for use according to the invention comprises an amino acid sequence having at least 99% 95%, or 97%, or 99%, or 100% sequence identity with SEQ ID NO: 4

An illustrative polynucleotide is SEQ ID NO: 5, which is the full nucleotide sequence of A232G, A237S, A238G, S240D, and D276N mutant, Hind III site (AAGCTT-in bold) and additional K and T amino acids. In some embodiments, the underlined portion of SEQ ID NO: 5, is omitted. The leader and additional nucleotides (Hind III site and K and T amino acids-for the addition of the amino acid sequence QASKT) are underlined.

atgattcaaaaacgaaagcggacagtttcgttcagacttgtgcttatgtgcacgctgttatttgtcag
tttgccgattacaaaaacatcagcgcaagcttccaagacggagatgaaagatgattttgcaaa
acttgaggaacaatttgatgcaaaactcgggatctttgcattggatacaggtacaaaccggacg
gtagcgtatcggccggatgagcgttttgcttttgcttcgacgattaaggctttaactgtaggcgtgctt
ttgcaacagaaatcaatagaagatctgaaccagagaataacatatacacgtgatgatcttgtaa
actacaacccgattacggaaaagcacgttgatacgggaatgacgctcaaagagcttgcggatg
cttcgcttcgatatagtgacaatgcggcacagaatctcattcttaaacaaattggcggacctgaaa
gtttgaaaaaggaactgaggaagattggtgatgaggttacaaatcccgaacgattcgaaccag
agttaaatgaagtgaatccgggtgaaactcaggataccagtacagcaagagcacttgtcacaa
gccttcgagcctttgctcttgaagataaacttccaagtgaaaaacgcgagcttttaatcgattggat
gaaacgaaataccactggagacgccttaatccgtgccggtgtgccggacggttgggaagtggg
tgataaaactggaagcggagattatggaacccggaatgacattgccatcatttggccgccaaa
aggagatcctgtcgttcttgcagtattatccagcagggataaaaaggacgccaagtatgataata
aacttattgcagaggcaacaaaggtggtaatgaaagccttaaacatgaacggcaaataa

In some embodiments, the polynucleotide has at least about 60% (*e.g*., about 60%, or about 61%, or about 62%, or about 63%, or about 64%, or about 65%, or about 66%, or about 67%, or about 68%, or about 69%, or about 70%, or about 71%, or about 72%, or about 73%, or about 74%, or about 75%, or about 76%, or about 77%, or about 78%, or about 79%, or about 80%, or about 81%, or about 82%, or about 83%, or about 84%, or about 85%, or about 86%, or about 87%, or about 88%, or about 89%, or about 90%, or about 91%, or about 92%, or about 93%, or about 94%, or about 95%, or about 96%, or about 97%, or about 98%, or about 99%) sequence identity with SEQ ID NO: 5 (with or without the underlined portion).

In various aspects, the beta-lactamase polypeptide has the sequence of SEQ ID NO: 6 (i.e., P2A) or is derived by one or more mutations of SEQ ID NO: 6:

ETGTISISQLNKNVWVHTELGYFNGEAVPSNGLVLNTSKGLVLVDSS
WDNKLTKELIEMVEKKFQKRVTDVIITHAHADRIGGITALKERGIKAHS
TALTAELAKNSGYEEPLGDLQTITSLKFGNTKVETFYPGKGHTEDNIV
VWLPQYQILAGGCLVKSAEAKDLGNVADAYVNEWSTSIENVLKRYG
NINSVVPGHGEVGDKGLLLHTLDLLK.

In some aspects of the invention, the beta-lactamase polypeptide for use according to the invention comprises an amino acid sequence having at least 95%, or 97%, or 99%, or 100% sequence identity with SEQ ID NO: 6. Additional sequences of beta-lactamases including P1A (i.e. SEQ ID NO: 1 except position 276 is D and not N), P2A, P3A/SYN-004, and P4A and derivatives thereof are described for example, in WO 2011/148041 and PCT/US2015/026457,

Further, the beta-lactamase polypeptide may include additional upstream residues from the first residue of SEQ ID NO: 1 (*see, e.g.,* JBC 258 (18): 11211, 1983, including the exo-large and exo-small versions of penP and penP1). Further, the beta-lactamase polypeptide may also include additional downstream residues from the last residue of SEQ ID NO: 1.

In some embodiments, the beta-lactamase includes one or more (*e.g.,* about 1, or about 2, or about 3, or about 4, or about 5, or about 6, or about 7, or about 8, or about 9, or about 10) mutations relative to SEQ ID NO: 1 or SEQ ID NO: 2. In some embodiments the beta-lactamase includes a variant of SYN-004, *e.g.,* a sequence with at least 95, 96, 97, 98, 99, 99.5, 99.8, 99.9% identity to SEQ ID NO: 1 or SEQ ID NO: 2. In various embodiments, one or more amino acid of SEQ ID NO: 1 is substituted with a naturally occurring amino acid, such as a hydrophilic amino acid (*e.g*., a polar and positively charged hydrophilic amino acid, such as arginine (R) or lysine (K); a polar and neutral of charge hydrophilic amino acid, such as asparagine (N), glutamine (Q), serine (S), threonine (T), proline (P), and cysteine (C), a polar and negatively charged hydrophilic amino acid, such as aspartate (D) or glutamate (E), or an aromatic, polar and positively charged hydrophilic amino acid, such as histidine (H)) or a hydrophobic amino acid (*e.g*., a hydrophobic, aliphatic amino acid such as glycine (G), alanine (A), leucine (L), isoleucine (I), methionine (M), or valine (V), a hydrophobic, aromatic amino acid, such as phenylalanine (F), tryptophan (W), or tyrosine (Y) or a non-classical amino acid (*e.g*., selenocysteine, pyrrolysine, *N*-formylmethionine β-alanine, GABA and δ-Aminolevulinic acid. 4-Aminobenzoic acid (PABA), D-isomers of the common amino acids, 2,4-diaminobutyric acid, α-amino isobutyric acid, 4-aminobutyric acid, Abu, 2-amino butyric acid, γ-Abu, ε-Ahx, 6-amino hexanoic acid, Aib, 2-amino isobutyric acid, 3-amino propionic acid, ornithine, norleucine, norvaline, hydroxyproline, sarcosme, citrulline, homocitrulline, cysteic acid, t-butylglycine, t-butylalanine, phenylglycine, cyclohexylalanine, β-alanine, fluoro-amino acids, designer amino acids such as β methyl amino acids, C α -methyl amino acids, N α-methyl amino acids, and amino acid analogs in general). In some embodiments, SEQ ID NO: 1 may have a Met and/or Thr preceding the first residue of the sequence. These residues may be similarly mutated as above.

Illustrative mutants include:

| **Mutations relative to P1A (based on the Ambler classification)** | **Name** |
|---|---|
| Wild type | RS310 (or P1A) |
| D276N | IS118 (or SYN-004) |
| I72S | IS222 |
| T160F | IS203 |
| R244T | IS217 |
| R244T D276K | IS215 |
| Q135M | IS197 |
| G156R A238T | IS235 |
| F33Y D276N | IS158 |
| F33Y S240P D276N | IS230 (or IS181) |
| F33Y A238T D276N | IS232 (or IS180) |
| I72S Q135M T160F (Block 1 mutants) | IS227 |
| A232G A237S A238G S240D (Block 2 mutants) | IS191 |
| A232G A237S A238G S240D R244T | IS229 |
| A232G A237S A238G S240D D276R | IS219 |
| A232G A237S A238G S240D D276K | IS221 |
| A232G A237S A238G S240D Q135M | IS224 |
| A238T | IS233 |
| T243I S266N D276N | IS234 (or IS176) |
| A232G A237S A238G S240D D276N | IS288 (or P4A) |

In all of these mutants, the numbering of residues corresponds to SEQ ID NO: 1. These residue numbers may be converted to Ambler numbers (Ambler et al., 1991, "A standard numbering scheme for the Class A β-lactamases," Biochem. J. 276:269-272, the contents of which are hereby incorporated by reference) through use of any conventional bioinformatic method, for example by using BLAST (Basic Local Alignment Search Tools) or FASTA (FAST-All).

In various embodiments, the beta-lactamase used in the invention is produced in bacterial cells such as an *E*. *coli* cell (*see, e.g.,* PCT/US15/47187, the entire contents of which are hereby incorporated by reference).

In various embodiments, the beta-lactamase for use according to the present invention hydrolyzes beta-lactams. Hydrolysis of the amide bond of the beta-lactam ring makes the antimicrobial agents biologically inactive. Examples of beta-lactam antibiotics will be apparent to one skilled in the art. Illustrative examples of intravenous beta-lactam antibiotics include, but are not limited to, penicillins (piperacillin/tazobactam), cephalosporins (cefepime), and/or carbapenems (meropenem; imipenem/cilastatin).

### Methods of Treatment

In some aspects, the present disclosure provides for compositions and methods for reducing the incidence and/or severity of complications associated with administration of IV beta-lactam antibiotics to allo-HCT recipients, including, but not limited to, aGVHD and/or VRE colonization and/or VRE bloodstream infection and/or infection or colonization, expansion of colonization, or monodomination by one or more multi-drug resistant pathogens.

In some embodiments, monodomination comprises about 25%, about 26%, about 27%, about 28%, about 29%, about 30%, about 31%, about 32%, about 33%, about 34%, or about 35% of microbiota within the subject's microbiome is a single organism.

In particular embodiments, the present disclosure provides for compositions and methods for preventing VRE colonization and/or VRE bloodstream infection. In some embodiments, methods prevent the gut dysbiosis associated with VRE colonization and/or VRE bloodstream infection.

In some aspects, a method of preventing VRE colonization and/or VRE bloodstream infection in a transplant recipient is provided that comprises administering the beta-lactamase agent of the invention. In some aspects, a method of preventing colonization and/or bloodstream infection of one or more multi-drug resistant organisms in a transplant recipient is provided that comprises administering the beta-lactamase agent.

In some embodiments, methods prevent the gut dysbiosis associated with colonization and/or bloodstream infection by the one or more multi-drug resistant organisms.

For example, in various embodiments, the present methods find use in the treatment or prevention of infections by one or more of the following pathogens: *Aeromonas hydrophila, Bacillus,* e.g., *Bacillus cereus, Bifidobacterium, Bordetella, Borrelia, Brucella, Burkholderia, C. difficile, Campylobacter,* e.g., *Campylobacter fetus* and *Campylobacter jejuni, Chlamydia, Chlamydophila, Clostridium,* e.g., *Clostridium botulinum, Clostridioides difficile* (formerly *Clostridium difficile*), and *Clostridium perfringens, Corynebacterium, Coxiella, Ehrlichia, Enterobacteriaceae,* e.g., Carbapenem-resistant *Enterobacteriaceae* (CRE) and Extended Spectrum Beta-Lactamase producing *Enterobacteriaceae* (ESBL-E), fluoroquinolone-resistant *Enterobacteriaceae, Enterococcus,* e.g., vancomycin-resistant *enterococcus spp.,* extended spectrum beta-lactam resistant *Enterococci* (ESBL), and Vancomycin-resistant *Enterococci* (*VRE*), *Escherichia,* e.g., enteroaggregative *Escherichia coli,* enterohemorrhagic *Escherichia coli,* enteroinvasive *Escherichia coli,* enteropathogenic *E. coli,* enterotoxigenic *Escherichia coli* (such as but not limited to LT and/or ST), *Escherichia coli* 0157:1-17, and multi-drug resistant bacteria *Escherichia coli, Francisella, Haemophilus, Helicobacter,* e.g., *Helicobacter pylori, Klebsiella,* e.g., *Klebsiellia pneumonia* and multi-drug resistant bacteria *Klebsiella, Legionella, Leptospira, Listeria,* e.g., *Lysteria monocytogenes, Morganella, Mycobacterium, Mycoplasma, Neisseria, Orientia, Plesiomonas shigelloides,* Antibiotic-resistant *Proteobacteria, Proteus, Pseudomonas, Rickettsia, Salmonella,* e.g., *Salmonella paratyphi, Salmonella spp.,* and *Salmonella typhi, Shigella,* e.g., *Shigella spp., Staphylococcus,* e.g., *Staphylococcus aureus* and *Staphylococcus spp., Streptococcus, Treponema, Vibrio,* e.g., *Vibrio cholerae, Vibrio parahaemolyticus, Vibrio spp.,* and *Vibrio vulnificus,* and *Yersinia,* e.g., *Yersinia enterocolitica.*

In various embodiments, the present methods find use in the treatment or prevention of infection an antibiotic- resistant bacterium, e.g., Antibiotic-resistant *Proteobacteria,* VRE, Carbapenem Resistant *Enterobacteriaceae* (CRE), fluoroquinolone-resistant *Enterobacteriaceae,* and Extended Spectrum Beta-Lactamase producing *Enterobacteriaceae* (ESBL-E).

In particular embodiments, the present disclosure provides for compositions and methods for reducing the incidence and/or severity of aGVHD. In various embodiments, the subject at risk for developing aGVHD is a transplant recipient. In further embodiments, the subject is a recipient of allo-HCT. In some embodiments, the subject is a recipient of one or more of bone marrow cells, peripheral blood cells, and umbilical cord cells. In various embodiments, the subject at risk for developing aGVHD is being administered or has been administered IV beta-lactam antibiotics.

In some embodiments, methods prevent the gut dysbiosis associated with aGVHD. Ir various embodiments, methods of the invention prevent the loss of gut microbiota diversity associated with aGVHD. For example, methods contemplate administration of compositions for modulating and/or reducing monodomination of any given bacterial strain (e.g., *Enterococcus*) associated with aGVHD. Further embodiments provide compositions and methods for preventing the decrease of microbiome diversity in subjects having undergone a transplant.

In some aspects, a method of reducing the incidence and/or severity of aGVHD in a transplant recipient is provided that comprises administering the beta-lactamase agent of the invention. For example, reducing the severity of aGVHD can include reducing the grade of aGVHD in patients receiving the beta-lactamase to a grade less than IV, which is categorized as the most severe form of aGVHD, or reducing the aGVHD by one or more grades (*e.g.,* grade III to grade II).

In embodiments, the present methods relate to reducing the incidence and/or severity of aGVHD in a transplant recipient. In embodiments, the transplant recipient is a cancer patient, *e.g.* one who has received or is receiving radiation or chemotherapy and subsequently has received allo-HCT. In embodiments, the transplant recipient has blood or bone marrow cancer. In embodiments, the transplant recipient is the recipient of a hematopoietic stem cell transplant. In certain embodiments, the cancer is selected from leukemia, lymphoma, myeloma, and myelodysplasia. In certain embodiments, the cancer is selected from osteosarcoma, Ewing tumors, chordomas, and chondrosarcomas.

aGVHD is the deterioration of cells or tissues that are transplanted from a donor to a recipient due to the recognition by the immune system of the recipient that the cells or tissues are foreign. Thus, because Class I MHC are on more cells of the body, it is most desirable to transplant cells and tissues from people that have highest matching Class I MHC profiles followed by the highest matching Class II MHC profiles. Thus, in most transplant recipients, aGVHD is due to activation of the immune system to mismatched Class II MHC molecules and other polymorphic proteins (minor histocompatibility antigens).

One option for treating cancers of the blood and bone marrow is to kill existing blood and marrow cells, e.g., through radiation or chemotherapy, and transplant similar cells from a healthy donor, referred to as an allogeneic hematopoietic stem cell transplant (allo-HCT).

In embodiments, the present methods relate to acute and chronic forms of GVHD. The classic acute or fulminant form of the disease (aGVHD) is typically observed within the first 100 days post-transplant and is a major challenge to the effectiveness of transplants owing to the associated morbidity and mortality. The chronic form of graft-versus-host-disease (cGVHD) traditionally occurs after 100 days. The appearance of moderate to severe cases of cGVHD adversely influences long-term survival. After bone marrow transplantation, T cells present in the graft, either as contaminants or intentionally introduced into the host, attack the tissues of the transplant recipient after perceiving host tissues as antigenically foreign. The T cells produce an excess of cytokines, including TNF alpha and interferon-gamma (IFNy). Tissue damage in cGVHD is primarily due to fibrosis. A wide range of host antigens can initiate graft-versus-host-disease, among them the human leukocyte antigens (HLAs). However, GVHD can occur even when HLA-identical siblings are the donors. aGVHD is characterized by selective damage to the liver, skin and mucosa, and the GI tract. Tissue damage in aGVHD is predominantly due to apoptosis. aGVHD severity is graded on a scale of I (mild) to IV (very severe) based on extent and type of lesion/rash (skin), diarrhea volume (GI), and serum bilirubin level (liver). cGVHD is characterized by a much broader tissue distribution than aGVHD. Skin and lungs are considered the primary target organs in cGVHD, along with the GI tract, liver, eyes, musculoskeletal system and hematopoietic system. A hyperacute and rapidly fatal form of aGVHD can occur within the first 2 weeks of allogeneic HCT, typically due to significant HLA mismatch or inadequate GVHD prophylaxis. Risk factors associated with cGVHD typically do not change after adjustment for prior aGVHD, suggesting that cGVHD is not simply an evolution of preceding aGVHD.

In embodiments, the present methods relate to reducing the incidence and/or severity of aGVHD. In embodiments, the present methods anticipate administration of the beta-lactamase to a patient who is treated with an IV beta-lactam antibiotic and has one or more risk factors of aGVHD, such as HLA "mismatch," or unrelated donor, older patient age, female donor to male recipient, intensity of the conditioning regimen or total body irradiation during conditioning regimen, and donor lymphocyte infusion. In embodiments, the present methods reduce the incidence and/or severity of aGVHD symptoms, such as skin rash, GI tract disorders, and liver symptoms.

In embodiments, the present methods relate to GVHD as defined by one of more of the Billingham Criteria: 1) administration of an immunocompetent graft, with viable and functional immune cells; 2) the recipient is immunologically histoincompatible; and 3) the recipient is immunocompromised and therefore cannot destroy or inactivate the transplanted cells.

### Formulations/Modified Release Profile

In various embodiments, the present disclosure employs modified release formulations comprising at least one beta-lactamase, wherein the formulation releases a substantial amount of the beta-lactamase into one or more regions of the GI tract. In some embodiments, the beta-lactamase is SYN-004 (a.k.a. P3A), or the other beta-lactamase agents described herein, or variants thereof (e.g., as described above). In some embodiments, the beta-lactamase is SYN-006 (a.k.a. P2A) or variants thereof. For example, the formulation may release at least about 60% of the beta-lactamase, for example, SYN-004 or SYN-006, after the stomach and into one or more regions of the GI tract.

In various embodiments, the modified-release formulations are designed for immediate release (*e.g.,* upon ingestion). In various embodiments, the modified-release formulations may have sustained-release profiles, *i.e.* slow release of the active ingredient(s) in the body (*e.g.,* GI tract) over an extended period of time. In various embodiments, the modified-release formulations may have a delayed-release profile, *i.e.* not immediately release the active ingredient(s) upon ingestion; rather, postponement of the release of the active ingredient(s) until the composition is lower in the GI tract; for example, for release in the small intestine (*e.g.,* one or more of duodenum, jejunum, ileum) or the large intestine (*e.g.,* one or more of cecum, ascending, transverse, descending or sigmoid portions of the colon, and rectum). For example, a composition can be enteric coated to delay release of the active ingredient(s) until it reaches the small intestine or large intestine. In some embodiments, there is not a substantial amount of the active ingredient(s) of the present formulations in the stool.

In various embodiments, the modified-release formulation releases at least 60% of the beta-lactamase (*e.g.,* SYN-004, or SYN-006, or the other beta-lactamase agents described herein, and variants thereof) after the stomach into one or more regions of the intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the intestine.

In various embodiments, the modified-release formulation releases at least 60% of the beta-lactamase (*e.g.,* SYN-004, or SYN-006, or the other beta-lactamase agents described herein, and variants thereof) in the small intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the small intestine.

In one embodiment, the modified-release formulation releases at least 60% of the beta-lactamase (*e.g.,* SYN-004, or SYN-006, or the other beta-lactamase agents described herein, and variants thereof) in the duodenum. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the duodenum.

In one embodiment, the modified-release formulation releases at least 60% of the beta-lactamase (*e.g.,* SYN-004, or SYN-006, or the other beta-lactamase agents described herein, and variants thereof) in the jejunum. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the jejunum.

In one embodiment, the modified-release formulation releases at least 60% of the beta-lactamase (*e.g.,* SYN-004, or SYN-006, or the other beta-lactamase agents described herein, and variants thereof) in the ileum and/or the ileocecal junction. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the ileum and/or the ileocecal junction.

In various embodiments, the modified-release formulation releases at least 60% of the beta-lactamase (*e.g.,* SYN-004, or SYN-006, or the other beta-lactamase agents described herein, and variants thereof) in the large intestine. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the large intestine.

In one embodiment, the modified-release formulation releases at least 60% of the beta-lactamase (*e.g.,* SYN-004, or SYN-006, or the other beta-lactamase agents described herein, and variants thereof) in the cecum. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the cecum.

In one embodiment, the modified-release formulation releases at least 60% of the beta-lactamase (*e.g.,* SYN-004, or SYN-006, or the other beta-lactamase agents described herein, and variants thereof) in the ascending colon. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the ascending colon.

In one embodiment, the modified-release formulation releases at least 60% of the beta-lactamase (*e.g.,* SYN-004, or SYN-006, or the other beta-lactamase agents described herein, and variants thereof) in the transverse colon. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the transverse colon.

In one embodiment, the modified-release formulation releases at least 60% of the beta-lactamase (*e.g.,* SYN-004, or SYN-006, or the other beta-lactamase agents described herein, and variants thereof) in the descending colon. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the descending colon.

In one embodiment, the modified-release formulation releases at least 60% of the beta-lactamase (*e.g.,* SYN-004, or SYN-006, or the other beta-lactamase agents described herein, and variants thereof) in the sigmoid colon. For example, the modified-release formulation releases at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% of the beta-lactamase (*e.g.,* SYN-004, or SYN-006, or the other beta-lactamase agents described herein, and variants thereof) in the sigmoid colon.

In various embodiments, the modified-release formulation does not substantially release the beta-lactamase (*e.g.,* SYN-004, or SYN-006, or the other beta-lactamase agents described herein, and variants thereof) in the stomach.

In certain embodiments, the modified-release formulation releases the beta-lactamase (*e.g.,* SYN-004, or SYN-006, or the other beta-lactamase agents described herein, and variants thereof) at a specific pH. For example, in some embodiments, the modified-release formulation is substantially stable in an acidic environment and substantially unstable (*e.g.,* dissolves rapidly or is physically unstable) in a near neutral to alkaline environment. In some embodiments, stability is indicative of not substantially releasing while instability is indicative of substantially releasing. For example, in some embodiments, the modified-release formulation is substantially stable at a pH of about 7.0 or less, or about 6.5 or less, or about 6.0 or less, or about 5.5 or less, or about 5.0 or less, or about 4.5 or less, or about 4.0 or less, or about 3.5 or less, or about 3.0 or less, or about 2.5 or less, or about 2.0 or less, or about 1.5 or less, or about 1.0 or less. In some embodiments, the present formulations are stable in lower pH areas and therefore do not substantially release in, for example, the stomach. In some embodiments, modified-release formulation is substantially stable at a pH of about 1 to about 4 or lower and substantially unstable at pH values that are greater. In these embodiments, the modified-release formulation is not substantially released in the stomach. In these embodiments, the modified-release formulation is substantially released in the small intestine (*e.g.,* one or more of the duodenum, jejunum, and ileum) and/or large intestine (*e.g.,* one or more of the cecum, ascending colon, transverse colon, descending colon, and sigmoid colon). In some embodiments, modified-release formulation is substantially stable at a pH of about 4 to about 5 or lower and consequentially is substantially unstable at pH values that are greater and therefore is not substantially released in the stomach and/or small intestine (*e.g.,* one or more of the duodenum, jejunum, and ileum). In these embodiments, the modified-release formulation is substantially released in the large intestine (*e.g.,* one or more of the cecum, ascending colon, transverse colon, descending colon, and sigmoid colon). In various embodiments, the pH values recited herein may be adjusted as known in the art to account for the state of the subject, *e.g.,* whether in a fasting or postprandial state.

In some embodiments, the modified-release formulation is substantially stable in gastric fluid and substantially unstable in intestinal fluid and, accordingly, is substantially released in the small intestine *(e.g.,* one or more of the duodenum, jejunum, and ileum) and/or large intestine *(e.g.,* one or more of the cecum, ascending colon, transverse colon, descending colon, and sigmoid colon).

In some embodiments, the modified-release formulation is stable in gastric fluid or stable in acidic environments. These modified-release formulations release about 30% or less by weight of the beta-lactamase *(e.g.,* SYN-004, or SYN-006, or the other beta-lactamase agents described herein, and variants thereof) and/or additional therapeutic agent in the modified-release formulation in gastric fluid with a pH of about 4 to about 5 or less, or simulated gastric fluid with a pH of about 4 to about 5 or less, in about 15, or about 30, or about 45, or about 60, or about 90 minutes. Modified-release formulations of the of the invention may release from about 0% to about 30%, from about 0% to about 25%, from about 0% to about 20%, from about 0% to about 15%, from about 0% to about 10%, about 5% to about 30%, from about 5% to about 25%, from about 5% to about 20%, from about 5% to about 15%, from about 5% to about 10% by weight of the beta-lactamase (*e.g.,* SYN-004, or SYN-006, or the other beta-lactamase agents described herein, and variants thereof) and/or additional therapeutic agent in the modified-release formulation in gastric fluid with a pH of 4-5, or less or simulated gastric fluid with a pH of 4-5 or less, in about 15, or about 30, or about 45, or about 60, or about 90 minutes. Modified-release formulations of the invention may release about 1%, about 2%, about 3%, about 4%, about 5%, about 6%, about 7%, about 8%, about 9%, or about 10% by weight of the total beta-lactamase *(e.g.,* SYN-004, or SYN-006, or the other beta-lactamase agents described herein, and variants thereof) and/or additional therapeutic agent in the modified-release formulation in gastric fluid with a pH of 5 or less, or simulated gastric fluid with a pH of 5 or less, in about 15, or about 30, or about 45, or about 60, or about 90 minutes.

In some embodiments, the modified-release formulation is unstable in intestinal fluid. These modified-release formulations release about 70% or more by weight of the beta-lactamase *(e.g.,* SYN-004, or SYN-006, or the other beta-lactamase agents described herein, and variants thereof) and/or additional therapeutic agent in the modified-release formulation in intestinal fluid or simulated intestinal fluid in about 15, or about 30, or about 45, or about 60, or about 90 minutes. In some embodiments, the modified-release formulation is unstable in near neutral to alkaline environments. These modified-release formulations release about 70% or more by weight of the beta-lactamase *(e.g.,* SYN-004, or SYN-006, or the other beta-lactamase agents described herein, and variants thereof) and/or additional therapeutic agent in the modified-release formulation in intestinal fluid with a pH of about 4-5 or greater, or simulated intestinal fluid with a pH of about 4-5 or greater, in about 15, or about 30, or about 45, or about 60, or about 90 minutes. A modified-release formulation that is unstable in near neutral or alkaline environments may release 70% or more by weight of beta-lactamase (*e.g.,* SYN-004, or SYN-006, or the other beta-lactamase agents described herein, and variants thereof) and/or additional therapeutic agent in the modified-release formulation in a fluid having a pH greater than about 5 *(e.g.,* a fluid having a pH of from about 5 to about 14, from about 6 to about 14, from about 7 to about 14, from about 8 to about 14, from about 9 to about 14, from about 10 to about 14, or from about 11 to about 14) in from about 5 minutes to about 90 minutes, or from about 10 minutes to about 90 minutes, or from about 15 minutes to about 90 minutes, or from about 20 minutes to about 90 minutes, or from about 25 minutes to about 90 minutes, or from about 30 minutes to about 90 minutes, or from about 5 minutes to about 60 minutes, or from about 10 minutes to about 60 minutes, or from about 15 minutes to about 60 minutes, or from about 20 minutes to about 60 minutes, or from about 25 minutes to about 90 minutes, or from about 30 minutes to about 60 minutes.

Examples of simulated gastric fluid and simulated intestinal fluid include, but are not limited to, those disclosed in the 2005 Pharmacopeia 23NF/28USP in Test Solutions at page 2858 and/or other simulated gastric fluids and simulated intestinal fluids known to those of skill in the art, for example, simulated gastric fluid and/or intestinal fluid prepared without enzymes.

In one embodiment, the modified-release formulation may remain essentially intact, or may be essentially insoluble, in gastric fluid. The modified-release formulation may include one or more delayed-release coatings that are pH dependent. Delayed-release coatings that are pH dependent will be substantially stable in acidic environments (pH of about 5 or less), and substantially unstable in near neutral to alkaline environments (pH greater than about 5). For example, the delayed-release coating may essentially disintegrate or dissolve in near neutral to alkaline environments such as are found in the small intestine (*e.g.,* one or more of the duodenum, jejunum, and ileum) and/or large intestine (*e.g.,* one or more of the cecum, ascending colon, transverse colon, descending colon, and sigmoid colon).

Alternatively, the stability of the modified-release formulation can be enzyme-dependent. In such embodiments, the modified-release formulation may include one or more delayed-release coatings that are enzyme- dependent. Delayed-release coating that are enzyme-dependent will be substantially stable in fluid that does not contain a particular enzyme and substantially unstable in fluid containing the enzyme. The delayed-release coating will essentially disintegrate or dissolve in fluid containing the appropriate enzyme. Enzyme-dependent control can be brought about, for example, by using materials which release the active ingredient only on exposure to enzymes in the intestine, such as galactomannans. Also, the stability of the modified-release formulation can be dependent on enzyme stability in the presence of a microbial enzyme present in the gut flora.

In various embodiments, the modified-release formulations comprising a beta-lactamase (*e.g.,* SYN-004, or SYN-006, or variants thereof) are substantially stable in chyme. For example, there is, in some embodiments, a loss of less about 50% or about 40%, or about 30%, or about 20%, or about 10% of beta-lactamase activity in about 10, or 9, or 8, or 7, or 6, or 5, or 4, or 3, or 2, or 1 hour from administration. In some embodiments, a dual pulse formulation is provided. In various embodiments, the present disclosure provides for modified-release formulations that release multiple doses of the beta-lactamase (*e.g.,* SYN-004, or SYN-006, or the other beta-lactamase agents described herein, and variants thereof), at different locations along the intestines, at different times, and/or at different pH. In an illustrative embodiment, the modified-release formulation comprises a first dose of the beta-lactamase and a second dose of the beta-lactamase, wherein the first dose and the second dose are released at different locations along the intestines, at different times, and/or at different pH. For example, the first dose is released at the duodenum, and the second dose is released at the ileum. In another example, the first dose is released at the jejunum, and the second dose is released at the ileum. In other embodiments, the first dose is released at a location along the small intestine (*e.g.,* the duodenum), while the second dose is released along the large intestine (*e.g.,* the ascending colon). In various embodiments, the modified-release formulation may release at least one dose, at least two doses, at least three doses, at least four doses, at least five doses, at least six doses, at least seven doses, or at least eight doses of the beta-lactamase (*e.g.,* SYN-004, or SYN-006, or the other beta-lactamase agents described herein, and variants thereof) at different locations along the intestines, at different times, and/or at different pH. Further the dual pulse description herein applies to modified-release formulations that release a beta-lactamase (*e.g.,* SYN-004, or SYN-006, or the other beta-lactamase agents described herein, and variants thereof) and an additional therapeutic agent.

In various embodiments, the present disclosure uses a modified-release formulation of beta-lactamase (*e.g.,* SYN-004, or SYN-006, or the other beta-lactamase agents described herein, and variants thereof) which may further comprise a pharmaceutically acceptable carrier or excipient. As one skilled in the art will recognize, the formulations can be in any suitable form appropriate for the desired use and route of administration.

In some embodiments, the administration of the modified-release formulation including beta-lactamase (and/or additional therapeutic agents) is any one of oral, intravenous, and parenteral. In some embodiments, the administration of the modified-release formulation including beta-lactamase (and/or additional agents) is not intravenous in order to, for example, prevent interference with an antibiotic administered systemically. In other embodiments, routes of administration include, for example: oral, intradermal, intramuscular, intraperitoneal, intravenous, subcutaneous, intranasal, epidural, sublingual, intranasal, intracerebral, intravaginal, transdermal, rectally, by inhalation, or topically, particularly to the ears, nose, eyes, or skin.

Any modified-release formulation including beta-lactamase (and/or additional therapeutic agents) as described herein can be administered orally. Such inventive formulations can also be administered by any other convenient route, for example, by intravenous infusion or bolus injection, by absorption through epithelial or mucocutaneous linings (*e.g.,* oral mucosa, rectal and intestinal mucosa, *etc*.) and can be administered together with an additional therapeutic agent. Administration can be systemic or local. In some embodiments, administration is not at the site of infection to avoid, for example, hydrolysis of an antibiotic at the site of infection. Various delivery systems are known, *e.g.,* encapsulation in liposomes, microparticles, microcapsules, capsules, etc., and can be used for administration. In specific embodiments, it may be desirable to administer locally to the area in need of treatment.

Suitable dosage forms for oral use include, for example, solid dosage forms such as tablets, dispersible powders, granules, and capsules. In one embodiment, the modified-release formulation is in the form of a capsule. In another embodiment, the modified-release formulation is in the form of a tablet. In yet another embodiment, the modified-release formulation is in the form of a soft-gel capsule. In a further embodiment, the modified-release formulation is in the form of a gelatin or hydroxypropyl methylcellulose (HPMC) capsule.

In some dosage forms, the agents described herein are mixed with at least one inert, pharmaceutically acceptable excipient or carrier such as sodium citrate, dicalcium phosphate, etc., and/or a) fillers or extenders such as starches, lactose, sucrose, glucose, mannitol, silicic acid, microcrystalline cellulose, and Bakers Special Sugar, *etc.,* b) binders such as, for example, carboxymethylcellulose, alginates, gelatin, polyvinylpyrrolidone, sucrose, acacia, polyvinyl alcohol, polyvinylpyrrolidone, methylcellulose, hydroxypropyl cellulose (HPC), and hydroxymethyl cellulose *etc.,* c) humectants such as glycerol, *etc.,* d) disintegrating agents such as agar-agar, calcium carbonate, potato or tapioca starch, alginic acid, certain silicates, sodium carbonate, cross-linked polymers such as crospovidone (cross-linked polyvinylpyrrolidone), croscarmellose sodium (cross-linked sodium carboxymethylcellulose), sodium starch glycolate, *etc.,* e) solution retarding agents such as paraffin, *etc.,* f) absorption accelerators such as quaternary ammonium compounds, *etc.,* g) wetting agents such as, for example, cetyl alcohol and glycerol monostearate, *etc.,* h) absorbents such as kaolin and bentonite clay, *etc.,* and i) lubricants such as talc, calcium stearate, magnesium stearate, solid polyethylene glycols, sodium lauryl sulfate, glyceryl behenate, *etc.,* and mixtures of such excipients. One of skill in the art will recognize that particular excipients may have two or more functions in the oral dosage form. In the case of an oral dosage form, for example, a capsule or a tablet, the dosage form may also comprise buffering agents.

The modified release formulation can additionally include a surface active agent. Surface active agents suitable for use in the present invention include, but are not limited to, any pharmaceutically acceptable, non-toxic surfactant. Classes of surfactants suitable for use in the compositions of the invention include, but are not limited to polyethoxylated fatty acids, PEG-fatty acid diesters, PEG-fatty acid mono- and diester mixtures, polyethylene glycol glycerol fatty acid esters, alcohol-oil transesterification products, polyglycerized fatty acids, propylene glycol fatty acid esters, mixtures of propylene glycol esters-glycerol esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-olyoxypropylene block copolymers, sorbitan fatty acid esters, lower alcohol fatty acid esters, ionic surfactants, and mixtures thereof. In some embodiments, compositions of the invention may comprise one or more surfactants including, but not limited to, sodium lauryl sulfate, polysorbate 20, polysorbate 40, polysorbate 60, polysorbate 80, and triethyl citrate.

The modified-release formulation can also contain pharmaceutically acceptable plasticizers to obtain the desired mechanical properties such as flexibility and hardness. Such plasticizers include, but are not limited to, triacetin, citric acid esters, triethyl citrate, phthalic acid esters, dibutyl sebacate, cetyl alcohol, polyethylene glycols, polysorbates or other plasticizers.

The modified-release formulation can also include one or more application solvents. Some of the more common solvents that can be used to apply, for example, a delayed-release coating composition include isopropyl alcohol, acetone, methylene chloride and the like.

The modified-release formulation can also include one or more alkaline materials. Alkaline material suitable for use in compositions of the invention include, but are not limited to, sodium, potassium, calcium, magnesium and aluminum salts of acids such as phosphoric acid, carbonic acid, citric acid and other aluminum/magnesium compounds. In addition the alkaline material may be selected from antacid materials such as aluminum hydroxides, calcium hydroxides, magnesium hydroxides and magnesium oxide.

The solid oral dosage forms can be prepared by, for example granulation (*e.g.,* wet or dry granulation) of the agents of the invention with one or more suitable excipients. Alternatively, the agents of the invention can be layered onto an inert core (*e.g.,* a nonpareil/sugar sphere such as a sucrose sphere or silica sphere) using conventional methods such as fluidized bed or pan coating, or extruded and spheronized using methods known in the art, into active compound-containing pellets. In embodiment, the beta-lactamase (*e.g.,* SYN-004, or SYN-006, or the other beta-lactamase agents described herein, and variants thereof) is spray-coated onto a sucrose sphere. Such pellets can then be incorporated into tablets or capsules using conventional methods.

Suspensions, in addition to the active agents, may contain suspending agents such as, for example, ethoxylated isostearyl alcohols, polyoxyethylene sorbitol and sorbitan esters, microcrystalline cellulose, aluminum metahydroxide, bentonite, agar-agar, tragacanth, *etc.,* and mixtures thereof.

Besides inert diluents, the oral compositions can also include adjuvants such as sweetening, flavoring, and perfuming agents.

Dosage forms suitable for parenteral administration (*e.g.,* intravenous, intramuscular, intraperitoneal, subcutaneous and intra-articular injection and infusion) include, for example, solutions, suspensions, dispersions, emulsions, and the like. They may also be manufactured in the form of sterile solid compositions (*e.g.,* lyophilized composition), which can be dissolved or suspended in sterile injectable medium immediately before use. They may contain, for example, suspending or dispersing agents known in the art.

The formulations comprising the beta-lactamase (and/or additional therapeutic agents) may conveniently be presented in unit dosage forms and may be prepared by any of the methods well known in the art of pharmacy. Such methods generally include the step of bringing the therapeutic agents into association with a carrier, which constitutes one or more accessory ingredients. Typically, the formulations are prepared by uniformly and intimately bringing the therapeutic agent into association with a liquid carrier (e.g., a phosphate-buffered saline), a finely divided solid carrier, or both, and then, if necessary, shaping the product into dosage forms of the desired formulation (*e.g.,* wet or dry granulation, powder blends, etc., followed by tableting using conventional methods known in the art).

In various embodiments, the modified-release formulation may utilize one or more modified-release coatings such as delayed-release coatings to provide for effective, delayed yet substantial delivery of the beta-lactamase (*e.g.,* SYN-004, or SYN-006, or the other beta-lactamase agents described herein, and variants thereof) to the GI tract together with, optionally, other additional therapeutic agents.

In one embodiment, the delayed-release coating includes an enteric agent that is substantially stable in acidic environments and substantially unstable in near neutral to alkaline environments. In an embodiment, the delayed-release coating contains an enteric agent that is substantially stable in gastric fluid. The enteric agent can be selected from, for example, solutions or dispersions of methacrylic acid copolymers, cellulose acetate phthalate, hydroxypropylmethyl cellulose phthalate, polyvinyl acetate phthalate, carboxymethylethylcellulose, and EUDRAGIT^{®}-type polymer (poly(methacrylic acid, methylmethacrylate), hydroxypropyl methylcellulose acetate succinate, cellulose acetate trimellitate, shellac or other suitable enteric coating polymers. The EUDRAGIT^{®}-type polymers include, for example, EUDRAGIT^{®} FS 30D, L 30 D-55, L 100-55, L 100, L 12,5, L 12,5 P, RL 30 D, RL PO, RL 100, RL 12,5, RS 30 D, RS PO, RS 100, RS 12,5, NE 30 D, NE 40 D, NM 30 D, S 100, S 12,5, and S 12,5 P. Similar polymers include Kollicoat^{®} MAE 30 DP and Kollicoat^{®} MAE 100 P. In some embodiments, one or more of EUDRAGIT^{®} FS 30D, L 30 D-55, L 100-55, L 100, L 12,5, L 12,5 P RL 30 D, RL PO, RL 100, RL 12,5, RS 30 D, RS PO, RS 100, RS 12,5, NE 30 D, NE 40 D, NM 30 D, S 100, S 12,5 S 12,5 P, Kollicoat^{®} MAE 30 DP and Kollicoat^{®} MAE 100 P is used. The following are incorporated by reference in their entireties: (1) Thakral et al., "Eudragit®: A technology evaluation" Expert Opinion on Drug Delivery Vol. 10 (2013) pp. 131-149; (2) Niranjan Patra et al., "Pharmaceutical significance of Eudragit: A review," Future Journal of Pharmaceutical Sciences Vol. 3 (10.1016/j.fjps.2017.02.001); (3) Sonje, Abhijit and Chandra, Amrish, "Comprehensive review on eudragit polymers," International Research Journal of Pharmacy Vol. 4 (10.7897/2230-8407.04515). In various embodiments, the enteric agent may be a combination of the foregoing solutions or dispersions. In an embodiment, the delayed-release coating includes the enteric agent EUDRAGIT^{®} L 30 D-55.

In certain embodiments, one or more coating system additives are used with the enteric agent. For example, one or more PlasACRYL^{™} additives may be used as an anti-tacking agent coating additive. Exemplary PlasACRYL^{™} additives include, but are not limited to PlasACRYL^{™} HTP20 and PlasACRYL^{™} T20. In an embodiment, PlasACRYL^{™} HTP20 is formulated with EUDRAGIT^{®} L 30 D-55 coatings. In another embodiment, PlasACRYL^{™} T20 is formulated with EUDRAGIT^{®} FS 30 D coatings.

In another embodiment, the delayed-release coating may degrade as a function of time when in aqueous solution without regard to the pH and/or presence of enzymes in the solution. Such a coating may comprise a water insoluble polymer. Its solubility in aqueous solution is therefore independent of the pH. The term "pH independent" as used herein means that the water permeability of the polymer and its ability to release pharmaceutical ingredients is not a function of pH and/or is only very slightly dependent on pH. Such coatings may be used to prepare, for example, sustained release formulations. Suitable water insoluble polymers include pharmaceutically acceptable non-toxic polymers that are substantially insoluble in aqueous media, *e.g.,* water, independent of the pH of the solution. Suitable polymers include, but are not limited to, cellulose ethers, cellulose esters, or cellulose ether-esters, *i.e.,* a cellulose derivative in which some of the hydroxy groups on the cellulose skeleton are substituted with alkyl groups and some are modified with alkanoyl groups. Examples include ethyl cellulose, acetyl cellulose, nitrocellulose, and the like. Other examples of insoluble polymers include, but are not limited to, lacquer, and acrylic and/or methacrylic ester polymers, polymers or copolymers of acrylate or methacrylate having a low quaternary ammonium content, or mixture thereof and the like. Other examples of insoluble polymers include EUDRAGIT RS^{®}, EUDRAGIT RL^{®}, and EUDRAGIT NE^{®}. Insoluble polymers useful in the present invention include polyvinyl esters, polyvinyl acetals, polyacrylic acid esters, butadiene styrene copolymers, and the like. In one embodiment, colonic delivery is achieved by use of a slowly-eroding wax plug (*e.g.,* various PEGS, including for example, PEG6000).

In a further embodiment, the delayed-release coating may be degraded by a microbial enzyme present in the gut flora. In one embodiment, the delayed-release coating may be degraded by a bacteria present in the small intestine. In another embodiment, the delayed-release coating may be degraded by a bacteria present in the large intestine.

In various embodiments, the disclosure provides a formulation comprising: a core particle having a base coat comprising one or more beta-lactamases (*e.g.,* SYN-004, or SYN-006, or the other beta-lactamase agents described herein, and variants thereof), and a delayed-release coating disposed over the coated core particle. The delayed-release coating may be substantially stable in acidic environments and/or gastric fluid, and/or substantially unstable in near neutral to alkaline environments or intestinal fluid thereby exposing the coated core particle to intestinal fluid. The base coat comprising one or more beta-lactamases may further comprise one or more additional therapeutic agents. Optionally a plurality of base coats may be applied to the core each of which may contain a beta-lactamase and/or an additional therapeutic agent. In an embodiment, the core particle includes sucrose. The formulation can be prepared by methods known in the art. For example, a beta-lactamases (*e.g.,* SYN-004, or SYN-006, or the other beta-lactamase agents described herein, and variants thereof) can be sprayed onto an inert core (*e.g.,* a sucrose core or sucrose sphere) and spray-dried with an enteric layer (*e.g.,* EUDRAGIT L30 D-55) to form beta-lactamase (*e.g.,* SYN-004, or SYN-006, or the other beta-lactamase agents described herein, and variants thereof)-containing pellets.

Optionally, the core particle may comprise one or more beta-lactamases (*e.g.,* SYN-004, or SYN-006, or the other beta-lactamase agents described herein, and variants thereof) and/or one or more additional therapeutic agents. In one embodiment, one or more doses of the beta-lactamase may be encapsulated in a core particle, for example, in the form of a microsphere. For example, the beta-lactamase may be combined with a polymer (*e.g.,* latex), and then formed into a particulate, micro-encapsulated enzyme preparation, without using a sucrose core. The microspheres thus formed may be optionally covered with a delayed-release coating.

A variety of approaches for generating particulates (such as microspheres, aggregates, other) are known which are amenable to the inclusion of enzymes. They typically involve at least two phases, one containing the enzyme, and one containing a polymer that forms the backbone of the particulate. Most common are coacervation, where the polymer is made to separate from its solvent phase by addition of a third component, or multiple phase emulsions, such as water in oil in water (w/o/w) emulsion where the inner water phase contains the protein, the intermediate organic phase contains the polymer, and the external water phase stabilizers that support the w/o/w double emulsion until the solvents can be removed to form the microspheres. Alternatively, the beta-lactamase (*e.g.,* SYN-004, or SYN-006, or the other beta-lactamase agents described herein, and variants thereof) and stabilizing excipients (for example, trehalose, mannitol, Tween 80, polyvinyl alcohol) are combined and sprayed from aqueous solution and collected. The particles are then suspended in a dry, water immiscible organic solvent containing polymer and release modifying compounds, and the suspension sonicated to disperse the particles. An additional approach uses aqueous phases but no organic solvent. Specifically, the enzyme, buffer components, a polymer latex, and stabilizing and release-modifying excipients are dissolved/dispersed in water. The aqueous dispersion is spray-dried, leading to coalescence of the latex, and incorporation of the protein and excipients in particles of the coalesced latex. When the release modifiers are insoluble at acidic conditions but soluble at higher pHs (such as carboxylic acid) then release from the matrix is inhibited in the gastric environment.

In some embodiments, before applying the delayed-release coating to the coated core particle the particle can optionally be covered with one or more separating layers comprising pharmaceutical excipients including alkaline compounds such as for instance pH-buffering compounds. The separating layer essentially separates the coated core particle from the delayed-release coating.

The separating layer can be applied to the coated core particle by coating or layering procedures typically used with coating equipment such as a coating pan, coating granulator or in a fluidized bed apparatus using water and/or organic solvents for the coating process. As an alternative the separating layer can be applied to the core material by using a powder coating technique. The materials for separating layers are pharmaceutically acceptable compounds such as, for instance, sugar, polyethylene glycol, polyvinylpyrrolidone, polyvinyl alcohol, polyvinyl acetate, hydroxypropyl cellulose, methyl-cellulose, ethylcellulose, hydroxypropyl methylcellulose, carboxymethylcellulose sodium and others, used alone or in mixtures. Additives such as plasticizers, colorants, pigments, fillers, anti-tacking and anti-static agents, such as for instance magnesium stearate, titanium dioxide, talc and other additives can also be included in the separating layer.

In some embodiments, the coated particles with the delayed-release coating may be further covered with an overcoat layer. The overcoat layer can be applied as described for the other coating compositions. The overcoat materials are pharmaceutically acceptable compounds such as sugar, polyethylene glycol, polyvinylpyrrolidone, polyvinyl alcohol, polyvinyl acetate, hydroxypropyl cellulose, methylcellulose, ethylcellulose, hydroxypropyl methylcellulose, carboxymethylcellulose sodium and others, used alone or in mixtures. The overcoat materials can prevent potential agglomeration of particles coated with the delayed-release coating, protect the delayed-release coating from cracking during the compaction process or enhance the tableting process.

In various embodiments, the formulation may comprise a plurality of modified-release particles or pellets or microspheres. In one embodiment, the formulation is in the form of capsules comprising multiple pellets. In one embodiment, the formulation is in the form of capsules comprising multiple microspheres. In some embodiments, the modified-release formulation is a capsule filled with a plurality of beta-lactamase-containing pellets (*e.g.,* SYN-004 or SYN-006, (or the other beta-lactamase agents described herein, and variants thereof)-containing pellets) from which the beta-lactamase is released. In an embodiment, the capsule is a gelatin capsule, such as a hard gelatin capsule. In another embodiment, the capsule is a hydroxypropyl methylcellulose (HPMC) capsule. For example, the formulation may be in the form of capsules comprising multiple pellets. For example, the formulation may be in the form of capsules such as, for example, gelatin or hydroxypropyl methylcellulose (HPMC) capsules comprising multiple enteric-coated pellets containing beta-lactamase (*e.g.,* SYN-004, or SYN-006, or the other beta-lactamase agents described herein, and variants thereof). In such an embodiment, a combination of pellets may be utilized in which each pellet is designed to release at a specific time point or location. In various embodiments, the pellets (*e.g.,* enteric-coated pellets) are designed to pass through the stomach unchanged and then release the beta-lactamase (*e.g.,* SYN-004, or SYN-006, or the other beta-lactamase agents described herein, and variants thereof) into one or more regions of the intestines. In some embodiments, the beta-lactamase-containing pellets may be enteric-coated to release the beta-lactamase (*e.g.,* SYN-004, or SYN-006, or the other beta-lactamase agents described herein, and variants thereof) at different intestinal pH values.

In various embodiments, the formulation is in the form of a capsule (*e.g.,* a hard gelatin or HPMC capsule) comprising a plurality of enteric-coated beta-lactamase-containing pellets. In such embodiments, the pellets (or each individual pellet) comprise a beta-lactamase (*e.g.,* SYN-004, or SYN-006, or the other beta-lactamase agents described herein, and variants thereof), a sucrose sphere, which the beta-lactamase, for example, SYN-004 or SYN-006, or a variant, is sprayed onto, a binder excipient (*e.g.,* hydroxypropylcellulose (HPC)), an enteric polymer (*e.g.,* EUDRAGIT L 30 D-55), a plasticizer (*e.g.,* triethyl citrate), a glidant *(e.g.,* glyceryl monostearate), an emulsifier, and buffer salts.

In various embodiments, the formulation is in the form of a capsule (*e.g*., a hard gelatin or HPMC capsule) comprising a plurality of enteric-coated beta-lactamase-containing pellets. In such embodiments, the pellets (or each individual pellet) comprise about 10-20% by weight of beta-lactamase (*e.g.,* SYN-004, or SYN-006, or the other beta-lactamase agents described herein, and variants thereof). For example, the beta-lactamase (*e.g.,* SYN-004, or SYN-006, or the other beta-lactamase agents described herein, and variants thereof) may be present at about 10%, about 11%, about 12%, about 13%, about 14%, about 15%, about 16%, about 17%, about 18%, about 19%, or about 20% by weight. In some embodiments, the pellets (or each individual pellet) comprise about 20-30% by weight sucrose sphere, which the beta-lactamase, for example, SYN-004 or SYN-006, or a variant, is sprayed onto. For example, the sucrose sphere may be present at about 20%, about 21%, about 22%, about 23%, about 24%, about 25%, about 26%, about 27%, about 28%, about 29%, or about 30% by weight. In various embodiments, the pellets (or each individual pellet) comprise about 30-40% by weight a binder excipient (*e.g.,* hydroxypropylcellulose (HPC)). For example, the binder excipient may be present at about 30%, about 31%, about 32%, about 33%, about 34%, about 35%, about 36%, about 37%, about 38%, about 39%, or about 40% by weight. In some embodiments, the pellets (or each individual pellet) comprise about 15-25% by weight an enteric polymer (*e.g.,* EUDRAGIT L 30 D-55). For example, the enteric polymer may be present at about 15%, about 16%, about 17%, about 18%, about 19%, about 20%, about 21%, about 22%, about 23%, about 24%, or about 25% by weight. In some embodiments, the pellets (or each individual pellet) comprise about 1.5- 2.5% by weight of plasticizer (*e.g.,* triethyl citrate). For example, the plasticizer may be present at about 1.5%, about 1.6%, about 1.7%, about 1.8%, about 1.9%, about 2%, about 2.1%, about 2.2%, about 2.3%, about 2.4%, about 2.5% by weight. In some embodiments, the pellets (or each individual pellet) comprise about 0.5-1.5% by weight glidant (*e.g.,* glyceryl monostearate). For example, the glidant may be present at about 0.5%, about 0.6%, about 0.7%, about 0.8%, about 0.9%, about 1%, about 1.1%, about 1.2%, about 1.3%, about 1.4%, or about 1.5% by weight. In some embodiments, the pellets (or each individual pellet) comprise about 0.1-1.0% by weight emulsifier (*e.g.,* polysorbate-80). For example, the emulsifier may be present at about 0.1%, about 0.2%, about 0.3%, about 0.4%, about 0.5%, about 0.6%, about 0.7%, about 0.8%, about 0.9%, or about 1% by weight. In some embodiments, the pellets (or each individual pellet) further comprise about 1-2% by weight buffer salts. For example, the buffer salts may be present at about 1.1%, about 1.2%, about 1.3%, about 1.4%, about 1.5%, about 1.6%, about 1.7%, about 1.8%, about 1.9%, or about 2% by weight. The weight as described herein refers to the total weight of all components excluding the weight of the capsule itself.

In some embodiments, the pellets (or each individual pellet) comprise about 16% by weight of the beta-lactamase (*e.g.,* SYN-004, or SYN-006, or the other beta-lactamase agents described herein, and variants thereof); about 23% by weight sucrose sphere; about 35% by weight a binder excipient (*e.g.,* hydroxypropylcellulose (HPC)); about 21% by weight an enteric polymer (*e.g.,* EUDRAGIT L 30 D-55); about 2% by weight of plasticizer (*e.g.,* triethyl citrate); about 1% by weight glidant (*e.g.,* glyceryl monostearate); about 0.5% by weight emulsifier (*e.g.,* polysorbate-80); and about 2% by weight buffer salts. The weight as described herein refers to the total weight of all components excluding the weight of the capsule itself.

For example, the pellets (or each individual pellet) comprise about 15.8% by weight of the beta-lactamase (*e.g.,* SYN-004, or SYN-006, or the other beta-lactamase agents described herein, and variants thereof); about 23.3% by weight sucrose sphere; about 35% by weight a binder excipient *(e.g.,* hydroxypropylcellulose (HPC)); about 20.8% by weight an enteric polymer (*e.g.,* EUDRAGIT L 30 D-55); about 2.1% by weight of plasticizer *(e.g.,* triethyl citrate); about 1.0% by weight glidant (*e.g.,* glyceryl monostearate); about 0.4% by weight emulsifier *(e.g.,* polysorbate-80); and about 1.6% by weight buffer salts. The weight as described herein refers to the total weight of all components excluding the weight of the capsule itself.

In various embodiments, the formulation is in the form of a capsule *(e.g.,* a hard gelatin or HPMC capsule) comprising about 75 mg of the beta-lactamase *(e.g.,* SYN-004, or SYN-006, or the other beta-lactamase agents described herein, and variants thereof). The capsule includes a plurality of enteric-coated beta-lactamase-containing pellets. In such embodiments, the formulation comprises about 10-20% by weight of the beta-lactamase (*e.g.,* SYN-004, or SYN-006, or the other beta-lactamase agents described herein, and variants thereof). For example, the beta-lactamase *(e.g.,* SYN-004, or SYN-006, or the other beta-lactamase agents described herein, and variants thereof) may be present at about 10%, about 11%, about 12%, about 13%, about 14%, about 15%, about 16%, about 17%, about 18%, about 19%, or about 20% by weight. In some embodiments, the formulation comprises about 15-25% by weight sucrose sphere. For example, the sucrose sphere may be present about 15%, about 16%, about 17%, about 18%, about 19%, about 20%, about 21%, about 22%, about 23%, about 24%, or about 25% by weight. In various embodiments, the formulation comprises about 25-35% by weight a binder excipient (*e.g.,* hydroxypropylcellulose (HPC)). For example, the binder excipient may be present at about 25%, about 26%, about 27%, about 28%, about 29%, about 30%, about 31%, about 32%, about 33%, about 34%, or about 35% by weight. In some embodiments, the formulation comprises about 10-25% by weight an enteric polymer (*e.g.,* EUDRAGIT L 30 D-55). For example, the enteric polymer may be present at about 10%, about 11%, about 12%, about 13%, about 14%, about 15%, about 16%, about 17%, about 18%, about 19%, about 20%, about 21%, about 22%, about 23%, about 24%, or about 25% by weight. In some embodiments, the formulation comprises about 1.5 - 2.5% by weight of plasticizer (*e.g.,* triethyl citrate). For example, the plasticizer may be present at about 1.5%, about 1.6%, about 1.7%, about 1.8%, about 1.9%, about 2%, about 2.1%, about 2.2%, about 2.3%, about 2.4%, about 2.5% by weight. In some embodiments, the formulation comprises about 0.5-1.5% by weight glidant (e.g., glyceryl monostearate). For example, the glidant may be present at about 0.5%, about 0.6%, about 0.7%, about 0.8%, about 0.9%, about 1%, about 1.1%, about 1.2%, about 1.3%, about 1.4%, or about 1.5% by weight. In some embodiments, the formulation comprises about 0.1-1.0% by weight emulsifier (e.g., polysorbate-80). For example, the emulsifier may be present at about 0.1%, about 0.2%, about 0.3%, about 0.4%, about 0.5%, about 0.6%, about 0.7%, about 0.8%, about 0.9%, or about 1% by weight. In some embodiments, the formulation comprises about 1-2% by weight buffer salts. For example, the buffer salts may be present at about 1%, about 1.1%, about 1.2%, about 1.3%, about 1.4%, about 1.5%, about 1.6%, about 1.7%, about 1.8%, about 1.9%, or about 2% by weight. In some embodiments, the formulation comprises about 10-20% by weight gelatin or HPMC capsule. For example, the gelatin or HPMC capsule may be about 10%, about 11%, about 12%, about 13%, about 14%, about 15%, about 16%, about 17%, about 18%, about 19%, or about 20% by weight.

In some embodiments, the formulation comprising about 75 mg of the beta-lactamase (*e.g.,* SYN-004, or SYN-006, or the other beta-lactamase agents described herein, and variants thereof). In such embodiments, the formulation comprises about 13% by weight of the beta-lactamase (*e.g.,* SYN-004, or SYN-006, or the other beta-lactamase agents described herein, and variants thereof); about 19% by weight sucrose sphere; about 29% by weight a binder excipient (*e.g.,* hydroxypropylcellulose (HPC)); about 17% by weight an enteric polymer (*e.g.,* EUDRAGIT L 30 D-55); about 2% by weight of plasticizer (*e.g.,* triethyl citrate); about 1% by weight glidant (*e.g.,* glyceryl monostearate); about 0.5% by weight emulsifier (*e.g.,* polysorbate-80); about 1% by weight buffer salts; and about 17% by weight gelatin or HPMC capsule.

For example, the formulation comprises about 13.1% by weight of the beta-lactamase (*e.g.,* SYN-004, or SYN-006, or the other beta-lactamase agents described herein, and variants thereof); about 19.4% by weight sucrose sphere; about 29.1% by weight a binder excipient (*e.g.,* hydroxypropylcellulose (HPC)); about 17.3% by weight an enteric polymer (*e.g.,* EUDRAGIT L 30 D-55); about 1.7% by weight of plasticizer (*e.g.,* triethyl citrate); about 0.9% by weight glidant (*e.g.,* glyceryl monostearate); about 0.4% by weight emulsifier *(e.g.,* polysorbate-80); about 1.3% by weight buffer salts; and about 16.8% by weight gelatin or HPMC capsule.

In various embodiments, the formulation is in the form of a capsule (*e.g.,* a hard gelatin or HPMC capsule) comprising about 25 mg of the beta-lactamase (*e.g.,* SYN-004, or SYN-006, or the other beta-lactamase agents described herein, and variants thereof). The capsule includes a plurality of enteric-coated beta-lactamase-containing pellets. In such embodiments, the formulation comprises about 5-15% by weight of the beta-lactamase (*e.g.,* SYN-004, or SYN-006, or the other beta-lactamase agents described herein, and variants thereof). For example, the beta-lactamase (*e.g.,* SYN-004, or SYN-006, or the other beta-lactamase agents described herein, and variants thereof) may be present at about 5%, about 6%, about 7%, about 8%, about 9%, about 10%, about 11%, about 12%, about 13%, about 14%, or about 15% by weight. In some embodiments, the formulation comprises about 10-20% by weight sucrose sphere. For example, the sucrose sphere may be present about 10%, about 11%, about 12%, about 13%, about 14%, about 15%, about 16%, about 17%, about 18%, about 19%, or about 20% by weight. In various embodiments, the formulation comprises about 15-25% by weight a binder excipient *(e.g.,* hydroxypropylcellulose (HPC)). For example, the binder excipient may be present at about 15%, about 16%, about 17%, about 18%, about 19%, about 20%, about 21%, about 22%, about 23%, about 24%, or about 25% by weight. In some embodiments, the formulation comprises about 10-20% by weight an enteric polymer *(e.g.,* EUDRAGIT L 30 D-55). For example, the enteric polymer may be present at about 10%, about 11%, about 12%, about 13%, about 14%, about 15%, about 16%, about 17%, about 18%, about 19%, or about 20% by weight. In some embodiments, the formulation comprises about 1.0 - 2.0% by weight of plasticizer *(e.g.,* triethyl citrate). For example, the plasticizer may be present at about 1.0%, about 1.1%, about 1.2%, about 1.3%, about 1.4%, about 1.5%, about 1.6%, about 1.7%, about 1.8%, about 1.9%, or about 2.0% by weight. In some embodiments, the formulation comprises about 0.1-1.0% by weight glidant (*e.g.,* glyceryl monostearate). For example, the glidant may be present at about 0.1%, about 0.2%, about 0.3%, about 0.4%, about 0.5%, about 0.6%, about 0.7%, about 0.8%, about 0.9%, or about 1% by weight. In some embodiments, the formulation comprises about 0.1-1.0% by weight emulsifier (*e.g.,* polysorbate-80). For example, the emulsifier may be present at about 0.1%, about 0.2%, about 0.3%, about 0.4%, about 0.5%, about 0.6%, about 0.7%, about 0.8%, about 0.9%, or about 1% by weight. In some embodiments, the formulation comprises about 0.5-1.5% by weight buffer salts. For example, the buffer salts may be present at about 0.5%, about 0.6%, about 0.7%, about 0.8%, about 0.9%, about 1.0%, about 1.1%, about 1.2%, about 1.3%, about 1.4%, or about 1.5% by weight. In some embodiments, the formulation comprises about 30-40% by weight gelatin or HPMC capsule. For example, the gelatin or HPMC capsule may be about 30%, about 31%, about 32%, about 33%, about 34%, about 35%, about 36%, about 37%, about 38%, about 39%, or about 40% by weight.

In some embodiments, the formulation comprising about 25 mg of the beta-lactamase (*e.g.,* SYN-004, or SYN-006, or the other beta-lactamase agents described herein, and variants thereof). In such embodiments, the formulation comprises about 10% by weight of the beta-lactamase (*e.g.,* SYN-004, or the other beta-lactamase agents described herein, and variants thereof); about 15% by weight sucrose sphere; about 22% by weight a binder excipient (*e.g.,* hydroxypropylcellulose (HPC)); about 13% by weight an enteric polymer (*e.g.,* EUDRAGIT L 30 D-55); about 1% by weight of plasticizer (*e.g.,* triethyl citrate); about 0.5% by weight glidant (*e.g.,* glyceryl monostearate); about 0.3% by weight emulsifier (*e.g.,* polysorbate-80); about 1% by weight buffer salts; and about 38% by weight gelatin or HPMC capsule.

For example, the formulation comprises about 9.8% by weight of the beta-lactamase (*e.g.,* SYN-004, or SYN-006, or the other beta-lactamase agents described herein, and variants thereof); about 14.5% by weight sucrose sphere; about 21.8% by weight a binder excipient (*e.g.,* hydroxypropylcellulose (HPC)); about 13% by weight an enteric polymer (*e.g.,* EUDRAGIT L 30 D-55); about 1.3% by weight of plasticizer (*e.g.,* triethyl citrate); about 0.6% by weight glidant (*e.g.,* glyceryl monostearate); about 0.3% by weight emulsifier (*e.g.,* polysorbate-80); about 1.0% by weight buffer salts; and about 37.7% by weight gelatin or HPMC capsule.

The present disclosure also provides for modified-release formulations that release multiple doses of the beta-lactamases (*e.g.,* SYN-004, or SYN-006, or the other beta-lactamase agents described herein, and variants thereof) and/or additional therapeutic agent along the GI tract. In such embodiments, the overall release profile of such a formulation may be adjusted by utilizing, for example, multiple particle types or multiple layers. In one embodiment, the first dose of the beta-lactamase may be formulated for release in, for example, the small intestine *(e.g.,* one or more of duodenum, jejunum, ileum) or the large intestine *(e.g.,* one or more of cecum, ascending, transverse, descending or sigmoid portions of the colon, and rectum), whereas the second dose is formulated for delayed release in, for example, a different region of the small intestine *(e.g.,* one or more of duodenum, jejunum, ileum) or the large intestine *(e.g.,* one or more of cecum, ascending, transverse, descending or sigmoid portions of the colon, and rectum). Alternatively, multiple doses are released at different locations along the intestine. For example, in one embodiment, the first dose of the beta-lactamase may be formulated for release in, for example, the small intestine *(e.g.,* one or more of duodenum, jejunum, ileum), whereas the second dose is formulated for delayed release in, for example, another part of the small intestine *(e.g.,* one or more of duodenum, jejunum, ileum). In another embodiment, the first dose of the beta-lactamase may be formulated for release in, for example, the large intestine *(e.g.,* one or more of cecum, ascending, transverse, descending or sigmoid portions of the colon, and rectum), whereas the second dose is formulated for delayed release in, for example, another part of the large intestine *(e.g.,* one or more of cecum, ascending, transverse, descending or sigmoid portions of the colon, and rectum).

In various embodiments, the agents described herein may be in the form of a pharmaceutically acceptable salt, namely those salts which are suitable for use in contact with the tissues of humans and other animals without undue toxicity, irritation, allergic response and the like, and are commensurate with a reasonable benefit/risk ratio. Pharmaceutically acceptable salts are well known in the art. The salts can be prepared *in situ* during the final isolation and purification of the therapeutic agents, or separately by reacting the free base function with a suitable acid or a free acid functionality with an appropriate alkaline moiety. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

In various embodiments, the present formulations provide a number of advantages. For instance, the inventors have successfully formulated a protein (i.e. beta-lactamase), which itself is challenging. This is compounded further by the GI tract environment in which the present formulations release drug in various embodiments. Further, in various embodiments, the present formulations provide for GI tract release that is sufficiently slow to allow good protective coverage in the GI tract from adverse effects of various antibiotics, e.g., in the small intestine (a benefit that is accentuated by an increase in beta-lactamase half-life that is commensurate with a slower release). Furthermore, by coating the drug substance layer of the present pellets with HPC, as opposed to EUDRAGIT, for example, the present formulations minimize the amount of EUGRAGIT in the formulations and therefore mitigate possible dose-limiting toxicity and manufacturing complications.

### Administration and Dosage

It will be appreciated that the actual dose of the beta-lactamase *(e.g.,* SYN-004, or SYN-006, or the other beta-lactamase agents described herein, and variants thereof) to be administered according to the present invention will vary according to, for example, the particular dosage form and the mode of administration. Many factors that may modify the action of the beta-lactamase (*e.g.,* body weight, gender, diet, time of administration, route of administration, rate of excretion, condition of the subject, drug combinations, genetic disposition and reaction sensitivities) can be taken into account by those skilled in the art. Administration can be carried out continuously or in one or more discrete doses within the maximum tolerated dose. Optimal administration rates for a given set of conditions can be ascertained by those skilled in the art using conventional dosage administration tests.

Individual doses of the beta-lactamase *(e.g.,* SYN-004, or SYN-006, or the other beta-lactamase agents described herein, and variants thereof) can be administered in unit dosage forms (*e.g.,* tablets or capsules) containing, for example, from about 0.01 mg to about 1,000 mg, from about 0.01 mg to about 950 mg, from about 0.01 mg to about 900 mg, from about 0.01 mg to about 850 mg, from about 0.01 mg to about 800 mg, from about 0.01 mg to about 750 mg, from about 0.01 mg to about 700 mg, from about 0.01 mg to about 650 mg, from about 0.01 mg to about 600 mg, from about 0.01 mg to about 550 mg, from about 0.01 mg to about 500 mg, from about 0.01 mg to about 450 mg, from about 0.01 mg to about 400 mg, from about 0.01 mg to about 350 mg, from about 0.01 mg to about 300 mg, from about 0.01 mg to about 250 mg, from about 0.01 mg to about 200 mg, from about 0.01 mg to about 150 mg, from about 0.01 mg to about 100 mg, from about 0.1 mg to about 90 mg, from about 0.1 mg to about 80 mg, from about 0.1 mg to about 70 mg, from about 0.1 mg to about 60 mg, from about 0.1 mg to about 50 mg, from about 0.1 mg to about 40 mg active ingredient, from about 0.1 mg to about 30 mg, from about 0.1 mg to about 20 mg, from about 0.1 mg to about 10 mg, from about 0.1 mg to about 5 mg, from about 0.1 mg to about 3 mg, from about 0.1 mg to about 1 mg per unit dosage form, or from about 5 mg to about 80 mg per unit dosage form. For example, a unit dosage form can be about 0.01 mg, about 0.02 mg, about 0.03 mg, about 0.04 mg, about 0.05 mg, about 0.06 mg, about 0.07 mg, about 0.08 mg, about 0.09 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, or about 1,000 mg, inclusive of all values and ranges therebetween. In an embodiment, individual dose of the beta-lactamase (*e.g.,* SYN-004, or SYN-006, or the other beta-lactamase agents described herein, and variants thereof) is administered in an unit dosage form containing 25 mg of the beta-lactamase. In another embodiment, individual dose of the beta-lactamase (*e.g.,* SYN-004, or SYN-006, or the other beta-lactamase agents described herein, and variants thereof) is administered in an unit dosage form containing 50 mg of the beta-lactamase. In a further embodiment, individual dose of the beta-lactamase (*e.g.,* SYN-004, or SYN-006, or the other beta-lactamase agents described herein, and variants thereof) is administered in an unit dosage form containing 75 mg of the beta-lactamase.

In one embodiment, the beta-lactamase is administered at an amount of from about 0.01 mg to about 100 mg daily, an amount of from about 0.01 mg to about 1,000 mg daily from about 0.01 mg to about 950 mg daily, from about 0.01 mg to about 900 mg daily, from about 0.01 mg to about 850 mg daily, from about 0.01 mg to about 800 mg daily, from about 0.01 mg to about 750 mg daily, from about 0.01 mg to about 700 mg daily, from about 0.01 mg to about 650 mg daily, from about 0.01 mg to about 600 mg daily, from about 0.01 mg to about 550 mg daily, from about 0.01 mg to about 500 mg daily, from about 0.01 mg to about 450 mg daily, from about 0.01 mg to about 400 mg daily, from about 0.01 mg to about 350 mg daily, from about 0.01 mg to about 300 mg daily, from about 0.01 mg to about 250 mg daily, from about 0.01 mg to about 200 mg daily, from about 0.01 mg to about 150 mg daily, from about 0.1 mg to about 100 mg daily, from about 0.1 mg to about 95 mg daily, from about 0.1 mg to about 90 mg daily, from about 0.1 mg to about 85 mg daily, from about 0.1 mg to about 80 mg daily, from about 0.1 mg to about 75 mg daily, from about 0.1 mg to about 70 mg daily, from about 0.1 mg to about 65 mg daily, from about 0.1 mg to about 60 mg daily, from about 0.1 mg to about 55 mg daily, from about 0.1 mg to about 50 mg daily, from about 0.1 mg to about 45 mg daily, from about 0.1 mg to about 40 mg daily, from about 0.1 mg to about 35 mg daily, from about 0.1 mg to about 30 mg daily, from about 0.1 mg to about 25 mg daily, from about 0.1 mg to about 20 mg daily, from about 0.1 mg to about 15 mg daily, from about 0.1 mg to about 10 mg daily, from about 0.1 mg to about 5 mg daily, from about 0.1 mg to about 3 mg daily, from about 0.1 mg to about 1 mg daily, or from about 5 mg to about 80 mg daily.

In various embodiments, the beta-lactamase is administered at a daily dose of about 0.01 mg, about 0.02 mg, about 0.03 mg, about 0.04 mg, about 0.05 mg, about 0.06 mg, about 0.07 mg, about 0.08 mg, about 0.09 mg, about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, or about 1,000 mg, inclusive of all values and ranges therebetween.

In some embodiments, a suitable dosage of the beta-lactamase (*e.g.,* SYN-004, or SYN-006, or the other beta-lactamase agents described herein, and variants thereof) is in a range of about 0.01 mg/kg to about 100 mg/kg of body weight of the subject, for example, about 0.01 mg/kg, about 0.02 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1 mg/kg, about 0.2 mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, about 1 mg/kg, about 1.1 mg/kg, about 1.2 mg/kg, about 1.3 mg/kg, about 1.4 mg/kg, about 1.5 mg/kg, about 1.6 mg/kg, about 1.7 mg/kg, about 1.8 mg/kg, 1.9 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, or about 100 mg/kg body weight, inclusive of all values and ranges therebetween. In other embodiments, a suitable dosage of the beta-lactamases in a range of about 0.01 mg/kg to about 10 mg/kg of body weight, in a range of about 0.01 mg/kg to about 9 mg/kg of body weight, in a range of about 0.01 mg/kg to about 8 mg/kg of body weight, in a range of about 0.01 mg/kg to about 7 mg/kg of body weight, in a range of 0.01 mg/kg to about 6 mg/kg of body weight, in a range of about 0.05 mg/kg to about 5 mg/kg of body weight, in a range of about 0.05 mg/kg to about 4 mg/kg of body weight, in a range of about 0.05 mg/kg to about 3 mg/kg of body weight, in a range of about 0.05 mg/kg to about 2 mg/kg of body weight, in a range of about 0.05 mg/kg to about 1.5 mg/kg of body weight, or in a range of about 0.05 mg/kg to about 1 mg/kg of body weight.

In various embodiments, the dose of SYN-004 or SYN-006 is between about 75 mg to about 300 mg, *e.g.,* about 75 mg, or about 100 mg, or about 125 mg, or about 150 mg, or about 175 mg, or about 200 mg, or about 225 mg, or about 250 mg, or about 275 mg, or about 300 mg.

In accordance with certain embodiments of the invention, the beta-lactamase may be administered, for example, about once per day, about every other day, about every third day, about once a week, about once every two weeks, about once every month, about once every two months, about once every three months, about once every six months, or about once every year. In certain embodiments, the beta-lactamase may be administered more than once daily, for example, about two times, about three times, about four times, about five times, about six times, about seven times, about eight times, about nine times, or about ten times daily.

### Additional Therapeutic Agents and Combination Therapy

Administration of the microbiome-protecting agents may be combined with additional therapeutic agents. Co-administration of the additional therapeutic agent and the present formulations may be simultaneous or sequential. Further the present formulations may comprise an additional therapeutic agent (*e.g.* via co-formulation).

In some embodiments, the modified-release formulations are administered ir combination with an additional therapeutic agent. In an embodiment, the additional therapeutic agent and the microbiome-protecting agent are combined into a single modified-release formulation. In some embodiments, the methods of treatment and/or prevention comprise administering the modified-release formulations of the present invention to a subject that is undergoing treatment with an additional therapeutic agent.

In one embodiment, the additional agent and the microbiome-protecting agent are administered to a subject simultaneously. The term "simultaneously" as used herein, means that the additional agent and the microbiome-protecting agent are administered with a time separation of no more than about 60 minutes, such as no more than about 30 minutes, no more than about 20 minutes, no more than about 10 minutes, no more than about 5 minutes, or no more than about 1 minute. Administration of the additional agent and the microbiome-protecting agent can be by simultaneous administration of a single formulation (*e.g.,* a formulation comprising the additional agent and the microbiome-protecting agent) or of separate formulations (*e.g.,* a first formulation including the additional agent and a second formulation including the microbiome-protecting agent).

Co-administration does not require the additional therapeutic agents to be administered simultaneously, if the timing of their administration is such that the pharmacological activities of the additional agent and the microbiome-protecting agent overlap in time, thereby exerting a combined therapeutic effect. For example, the additional agent and the microbiome-protecting agent can be administered sequentially. The term "sequentially" as used herein means that the additional agent and the microbiome-protecting agent are administered with a time separation of more than about 60 minutes. For example, the time between the sequential administration of the additional agent and the microbiome-protecting agent can be more than about 60 minutes, more than about 2 hours, more than about 5 hours, more than about 10 hours, more than about 1 day, more than about 2 days, more than about 3 days, or more than about 1 week apart. The optimal administration times will depend on the rates of metabolism, excretion, and/or the pharmacodynamic activity of the additional agent and the microbiome-protecting agent being administered. Either the additional agent or the microbiome-protecting agent may be administered first.

In a further embodiment, the additional therapeutic agent and the microbiome-protecting agent are administered to a subject simultaneously but the release of additional therapeutic agent and the microbiome-protecting agent from their respective dosage forms (or single unit dosage form if co-formulated) in the GI tract occurs sequentially.

Co-administration also does not require the additional therapeutic agents to be administered to the subject by the same route of administration. Rather, each therapeutic agent can be administered by any appropriate route, for example, parenterally or non-parenterally.

In some embodiments, the one or more microbiome-protecting agent is used in a method of reducing the incidence and/or severity of complications associated with administration of IV beta-lactam antibiotics to allo-HCT recipients, such aGVHD and VRE colonization and/or VRE bloodstream infection.

In some embodiments, the one or more microbiome-protecting agent is used in combination with one or more therapeutic agents that prevent or treat one or more symptoms or side effects of a disorder associated with allo-HCT, such as aGVHD and VRE colonization and/or VRE bloodstream infection. Such agents include, but are not limited to, immunosuppression agents, such as corticosteroids (such as methylprednisolone or prednisone) and other immunosuppressive drugs. An illustrative aGVHD treatment, in some embodiments, is prednisone. Other illustrative aGVHD preventatives and treatments include ibrutinib (*e.g.* IMBRUVICA), mycophenolate mofetil, mTOR inhibitors, such as sirolimus (rapamycin), everolimus, calcineurin inhibitors, such as tacrolimus or cyclosporine, ciclosporin, monoclonal antibodies such as infliximab (*e.g.* REMICADE), tocilizumab (*e.g.* ACTEMRA), alemtuzumab (*e.g.* CAMPATH), basiliximab (*e.g.* SIMULECT), daclizumab (*e.g.* ZINBRYTA), and denileukin diftitox (*e.g.* ONTAK), antithymocyte globulin (ATG), anti-lymphocyte globulin (ALG), pentostatin (*e.g.* NIPENT), ruxolitinib (*e.g.* JAKAFI), and photopheresis.

In some embodiments, reducing the incidence and/or severity of aGVHD by administration of a beta-lactamase enables a reduction in the dose, length of dosing, or dosing frequency of additional therapies used to prevent or treat aGVHD including methylprednisolone or prednisone and other immunosuppressive drugs, including ibrutinib (*e.g.* IMBRUVICA), mycophenolate mofetil, mTOR inhibitors, such as sirolimus (rapamycin), everolimus, calcineurin inhibitors, such as tacrolimus or cyclosporine, ciclosporin, monoclonal antibodies such as infliximab (*e.g.* REMICADE), tocilizumab (e.g. ACTEMRA), alemtuzumab (*e.g.* CAMPATH), basiliximab (*e.g.* SIMULECT), daclizumab (*e.g.* ZINBRYTA), and denileukin diftitox (*e.g.* ONTAK), antithymocyte globulin (ATG), anti-lymphocyte globulin (ALG), pentostatin (e.g. NIPENT), ruxolitinib (*e.g.* JAKAFI), and photopheresis. For instance, this may spare the patient therapy with one or more steroids at a higher dosing or frequency.

### Kits

The disclosure provides kits that can simplify the administration of the modified-release formulation described herein. The kit is an assemblage of materials or components, including at least one of the modified-release formulations described herein. The exact nature of the components configured in the kit depends on its intended purpose. In one embodiment, the kit is configured for the purpose of treating human subjects.

Instructions for use may be included in the kit. Instructions for use typically include a tangible expression describing the technique to be employed in using the components of the kit to affect a desired outcome, such as to treat a disorder associated described herein. Optionally, the kit also contains other useful components, such as, diluents, buffers, pharmaceutically acceptable carriers, syringes, catheters, applicators, pipetting or measuring tools, bandaging materials or other useful paraphernalia as will be readily recognized by those of skill in the art.

The materials and components assembled in the kit can be provided to the practitioner store in any convenience and suitable ways that preserve their operability and utility. For example, the components can be provided at room, refrigerated or frozen temperatures. The components are typically contained in suitable packaging materials. In various embodiments, the packaging material is constructed by well-known methods, preferably to provide a sterile, contaminant-free environment. The packaging material may have an external label which indicates the contents and/or purpose of the kit and/or its components.

### Definitions

As used herein, "a," "an," or "the" can mean one or more than one.

Further, the term "about" when used in connection with a referenced numeric indication means the referenced numeric indication plus or minus up to 10% of that referenced numeric indication. For example, the language "about 50%" covers the range of 45% to 55%.

An "effective amount," when used in connection with medical uses is an amount that is effective for providing a measurable treatment, prevention, or reduction in the rate of pathogenesis of a disorder of interest.

As used herein, something is "decreased" if a read-out of activity and/or effect is reduced by a significant amount, such as by at least about 10%, at least about 20%, at least about 30%, at least about 40%, at least about 50%, at least about 60%, at least about 70%, at least about 80%, at least about 90%, at least about 95%, at least about 97%, at least about 98%, or more, up to and including at least about 100%, in the presence of an agent or stimulus relative to the absence of such modulation. As will be understood by one of ordinary skill in the art, in some embodiments, activity is decreased and some downstream read-outs will decrease but others can increase.

Conversely, activity is "increased" if a read-out of activity and/or effect is increased by a significant amount, for example by at least about 10%, at least about 20%, at least about 30%, at least about 40%, at least about 50%, at least about 60%, at least about 70%, at least about 80%, at least about 90%, at least about 95%, at least about 97%, at least about 98%, or more, up to and including at least about 100% or more, at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold, in the presence of an agent or stimulus, relative to the absence of such agent or stimulus.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the compositions and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of terms such as including, containing, or having, is used herein to describe and claim the invention, the present invention, or embodiments thereof, may alternatively be described using alternative terms such as "consisting of' or "consisting essentially of."

As used herein, the words "preferred" and "preferably" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

The amount of compositions described herein needed for achieving a therapeutic effect may be determined empirically in accordance with conventional procedures for the particular purpose. Generally, for administering therapeutic agents (*e.g.,* beta-lactamases and/or additional therapeutic agents described herein) for therapeutic purposes, the therapeutic agents are given at a pharmacologically effective dose. A "pharmacologically effective amount," "pharmacologically effective dose," "therapeutically effective amount," or "effective amount" refers to an amount sufficient to produce the desired physiological effect or amount capable of achieving the desired result, particularly for treating the disorder or disease. An effective amount as used herein would include an amount sufficient to, for example, delay the development of a symptom of the disorder or disease, alter the course of a symptom of the disorder or disease *(e.g.,* slow the progression of a symptom of the disease), reduce or eliminate one or more symptoms or manifestations of the disorder or disease, and reverse a symptom of a disorder or disease. Therapeutic benefit also includes halting or slowing the progression of the underlying disease or disorder, regardless of whether improvement is realized.

Effective amounts, toxicity, and therapeutic efficacy can be determined by standard pharmaceutical procedures in cell cultures, tissue samples, tissue homogenates or experimental animals, *e.g.,* for determining the LD50 (the dose lethal to about 50% of the population) and the ED50 (the dose therapeutically effective in about 50% of the population). The dosage can vary depending upon the dosage form employed and the route of administration utilized. The dose ratio between toxic and therapeutic effects is the therapeutic index and can be expressed as the ratio LD50/ED50. In some embodiments, compositions and methods that exhibit large therapeutic indices are preferred. A therapeutically effective dose can be estimated initially from in vitro assays, including, for example, cell culture assays. Also, a dose can be formulated in animal models to achieve a circulating plasma concentration range that includes the IC50 as determined in cell culture, or in an appropriate animal model. Levels of the described compositions in plasma can be measured, for example, by high performance liquid chromatography. The effects of any particular dosage can be monitored by a suitable bioassay. The dosage can be determined by a physician and adjusted, as necessary, to suit observed effects of the treatment.

In certain embodiments, the effect will result in a quantifiable change of at least about 10%, at least about 20%, at least about 30%, at least about 50%, at least about 70%, or at least about 90%. In some embodiments, the effect will result in a quantifiable change of about 10%, about 20%, about 30%, about 50%, about 70%, or even about 90% or more. Therapeutic benefit also includes halting or slowing the progression of the underlying disease or disorder, regardless of whether improvement is realized.

As used herein, "methods of treatment" are equally applicable to use of a composition for treating the diseases or disorders described herein and/or compositions for use and/or uses in the manufacture of a medicaments for treating the diseases or disorders described herein.

### EXAMPLES

### Example 1: SYN-004 (P3A) degraded and/or reduced piperacillin levels in mouse stool

The purpose of this experiment was to determine whether a liquid formulation of SYN-004 (e.g., a beta-lactamase in a phosphate-buffered saline solution) could be delivered to the small and large intestine of mice via oral gavage.

Mice received a subcutaneous administration of 500 mg/kg piperacillin/tazobactam twice per day for two days. The mice were concurrently divided into two cohorts: (1) a first cohort that received subcutaneous administration of 500 mg/kg piperacillin/tazobactam twice per day for two days but did not receive a liquid formulation of P3A (e.g., a beta-lactamase in saline solution) via oral gavage; and (2) a second cohort that received subcutaneous administration of 500 mg/kg piperacillin/tazobactam twice per day for two days and administration of P3A via oral gavage at 10 mg/kg twice per day for two days. A control cohort of mice received neither piperacillin/tazobactam nor P3A via oral gavage.

After 2 days, mouse stool was evaluated for presence of piperacillin. **Figure 1** shows that the amount of piperacillin (µg/g stool) was reduced in mice that received the subcutaneous administration of 500 mg/kg piperacillin/tazobactam twice per day for two days + a liquid formulation of P3A (e.g., a beta-lactamase in a phosphate-buffered saline solution) via oral gavage twice per day for two days. The leftmost bar of the bar graph corresponds to data from the control mouse cohort; the middle bar of the bar graph corresponds to data from the first cohort of mice that received piperacillin/tazobactam but did not receive P3A; and the rightmost bar of the bar graph corresponds to data from the second cohort of mice that received both piperacillin/tazobactam and P3A.

### Example 2: SYN-004 (P3A) protected Firmicutes bacterial phylum from piperacillin/tazobactam

Mice received a subcutaneous administration of 125 mg/kg piperacillin/tazobactam twice per day for two days. The mice were concurrently divided into two cohorts: (1) a first cohort that received subcutaneous administration of 125 mg/kg piperacillin/tazobactam twice per day for two days but did not receive a liquid formulation of P3A (e.g., a beta-lactamase in a phosphate-buffered saline solution) via oral gavage; and (2) a second cohort that received subcutaneous administration of 125 mg/kg piperacillin/tazobactam twice per day for two days and administration of P3A via oral gavage at 10 mg/kg twice per day for two days. A control cohort of mice received neither piperacillin/tazobactam nor P3A via oral gavage. Another control cohort of mice received P3A only.

After two days, 16S sequencing analysis of mouse stool was performed in order to assess the level of *Firmicutes* bacterial phylum genera (*e.g*., G+ anaerobes including *Lactobacillus* and *Clostridium* that produce short chain fatty acids) in the mouse stool. **Figure 2** shows that the abundance of *Firmicutes* in the mouse stool was reduced by piperacillin/tazobactam alone and the fold abundance of *Firmicutes* was recovered and protected in the presence of P3A. The leftmost bar of the bar graph corresponds to data from the control mouse cohort, where no drug or beta-lactamase was administered; the bar second to the left corresponds to data from the cohort of mice that received P3A only; the bar second to the right corresponds to data from the first cohort of mice that received piperacillin/tazobactam but did not receive a liquid formulation of P3A (e.g., a beta-lactamase in a phosphate-buffered saline solution); and the rightmost bar of the bar graph corresponds to data from the second cohort of mice that received both piperacillin/tazobactam and P3A.

16S sequencing analysis was also performed to evaluate the total bacteria copies present per gram of mouse stool. **Figure 3** shows that the total bacterial copies present in the mouse stool were decreased by piperacillin/tazobactam and this decrease was reduced when piperacillin/tazobactam was administered with P3A. The leftmost bar of the bar graph corresponds to data from the control mouse cohort, where no drug or beta-lactamase was administered; the bar second to the left corresponds to data from the cohort of mice that received P3A only; the bar second to the right corresponds to data from the first cohort of mice that received piperacillin/tazobactam but did not receive a liquid formulation of P3A (e.g., a beta-lactamase in a phosphate-buffered saline solution); and the rightmost bar of the bar graph corresponds to data from the second cohort of mice that received both piperacillin/tazobactam and P3A.

### Example 3: SYN-004 (P3A) reduced antibiotic-exacerbated GVHD in model organism

This experiment was conducted to assess the effect of SYN-004 administration on protecting and/or reducing GVHD severity associated with bone marrow transplantation.

129S1 mice were lethally irradiated and then transplanted with bone marrow cells (B6 donor bone marrow) at 5M and defined doses of splenic T lymphocytes from C57BL/6 mice at 2M. Both of these mice share the MHC-type H2^{b} and thus represent a clinically relevant MHC-matched transplantation model.

The mice were divided into four cohorts: (1) a cohort that received B6 donor bone marrow cells at 5M only; (2) a cohort that received B6 donor bone marrow cells at 5M + T cells at 2M + subcutaneous administration of 100 mg/kg piperacillin/tazobactam for three times per week starting at day 10 post-bone marrow transplantation +a liquid formulation of P3A (e.g., a beta-lactamase in a phosphate-buffered saline solution) via oral gavage at 20 mg/kg for three times per week starting at day 10 post-bone marrow transplantation and at the same time as the piperacillin/tazobactam administration; (3) a cohort that received B6 donor bone marrow cells + T cells; and (4) a cohort that received B6 donor bone marrow cells at 5M + T cells at 2M + subcutaneous administration of 100 mg/kg piperacillin/tazobactam for three times per week starting at day 10 post-bone marrow transplantation.

**Figure 4** shows the results of the survival study with an endpoint of 30 days post-bone marrow transplantation. The data shows that P3A administration reduced antibiotic-exacerbated GVHD associated with bone marrow transplantation in terms of reducing mortality.

### Example 4: SYN-004 (P3A) prevented loss of gut microbiota diversity and intestinal dysbiosis in model organism

This experiment was conducted to assess the effect of SYN-004 administration on modulating and/or reducing *Enterococcus* monodomination. The experiment was also designed to assess whether SYN-004 administration could prevent the decrease of microbiome diversity in lethally irradiated mice having undergone a bone marrow transplantation.

129S1 mice were lethally irradiated and then transplanted with bone marrow cells (B6 donor bone marrow) at 5M and defined doses of splenic T lymphocytes from C57BL/6 mice at 2M. The mice were divided in three cohorts: (1) a cohort that received administration of saline; (2) a cohort that received administration of piperacillin/tazobactam; and (3) a cohort that received administration of piperacillin/tazobactam (Zosyn, abbreviated as ZO) and SYN-004 (P3A; abbreviated as Blact).

Stool samples were collected at day 21 post-transplantation and 16S sequencing analysis was performed. **Figure 5** and **Figure 6** depict the results of the 16S sequencing analysis, where stool samples from mice having been administered both piperacillin/tazobactam and SYN-004 showed reduced *Enterococcus* monodominance, as compared to stool samples from mice having been administered piperacillin/tazobactam only. With regards to Figure 6, empty bars indicate that that particular sample did not amplify.

### Example 5: SYN-006 (P2A) protected model organism's gut microbiome from beta-lactam antibiotics

Experiments were conducted to determine whether a liquid formulation of SYN-006 (e.g., a beta-lactamase in a phosphate-buffered saline solution) could be delivered to the small and large intestine of mice via oral gavage.

Mice received a subcutaneous administration of 100 mg/kg imipenem+cilastatin for two days, with stool samples collected on the third day. The mice were concurrently divided into four cohorts: (1) a first cohort that received subcutaneous administration of 100 mg/kg imipenem+cilastatin for two days but did not receive a liquid formulation of P2A (e.g., a beta-lactamase in saline solution) via oral gavage; (2) a second cohort that received subcutaneous administration of 100 mg/kg imipenem+cilastatin for two days and administration of P2A via oral gavage at 1 mg/kg twice per day for two days; (3) a third cohort that received subcutaneous administration of 100 mg/kg imipenem+cilastatin for two days and administration of P2A via oral gavage at 10 mg/kg twice per day for two days; and (4) a fourth cohort that received subcutaneous administration of 100 mg/kg imipenem+cilastatin for two days and administration of P2A via oral gavage at 50 mg/kg twice per day for two days.

On day 3, mouse stool was collected. 16S sequencing analysis of mouse stool was performed in order to assess the level of *Firmicutes* bacterial phylum genera (*e.g.*, G+ anaerobes including *Lactobacillus* and *Clostridium* that produce short chain fatty acids) in the mouse stool. **Figure 7** shows that the fold abundance of Firmicutes was recovered and protected in the presence of both imipenem+cilastatin and 10 mg/kg and 50 mg/kg doses of P2A, as compared to in the presence of imipenem+cilastatin alone. The leftmost bar of the bar graph corresponds to data from the cohort of mice that received imipenem+cilastatin only; the bar second from the left corresponds to data from the second cohort of mice that received imipenem+cilastatin and P2A at 1 mg/kg; the bar third from the left corresponds to data from the third cohort of mice that received imipenem+cilastatin and P2A at 10 mg/kg; and the rightmost bar of the bar graph corresponds to data from the fourth cohort of mice that received imipenem+cilastatin and P2A at 50 mg/kg.

Next, experiments were conducted to assess the effect of SYN-006 (P2A) administration on modulating and/or reducing *Enterococcus* monodomination, and specifically, overgrowth of *Enterococcus faecium,* in imipenem-treated mice.

Mice received a subcutaneous administration of 100 mg/kg imipenem+cilastatin for two days, with stool samples collected on the third day. The mice were concurrently divided into four cohorts: (1) a first cohort that received subcutaneous administration of 100 mg/kg imipenem+cilastatin for two days but did not receive a liquid formulation of P2A (e.g., a beta-lactamase in saline solution) via oral gavage; (2) a second cohort that received subcutaneous administration of 100 mg/kg imipenem+cilastatin for two days and administration of P2A via oral gavage at 1 mg/kg twice per day for two days; (3) a third cohort that received subcutaneous administration of 100 mg/kg imipenem+cilastatin for two days and administration of P2A via oral gavage at 10 mg/kg twice per day for two days; and (4) a fourth cohort that received subcutaneous administration of 100 mg/kg imipenem+cilastatin for two days and administration of P2A via oral gavage at 50 mg/kg twice per day for two days. Stool samples were collected on day 3 and 16S sequencing analysis was performed. **Figure 8** depicts the results of the 16S sequencing analysis, where stool samples from mice administered both imipenem+cilastatin and SYN-006 (at 10 mg/kg and 50 mg/kg doses) showed reduced *Enterococcus* monodominance compared to stool samples from mice having been administered imipenem+cilastatin only. **Figure 9** shows the relative abundance of *Enterococcus faecium* in mouse stool when treated with imipenem+cilastatin alone or in combination with P2A at doses of 1 mg/kg, 10 mg/kg, and 50 mg/kg. The results show that SYN-006 (P2A) prevents overgrowth of *Enterococcus faecium* in imipenem-treated mice. In each histogram, the clusters on the left represent pre-treatment values and the clusters on the right represent post-treatment values.

### Example 6: SYN-006 (P2A) reduced antibiotic-exacerbated GVHD in model organism

This experiment was conducted to assess the effect of SYN-006 (P2A) administration on protecting and/or reducing GVHD severity associated with bone marrow transplantation.

129S1 mice were lethally irradiated and then transplanted with bone marrow cells (B6 donor bone marrow) at 5M and defined doses of splenic T lymphocytes from C57BL/6 mice at 2M. Both of these mice share the MHC-type H2^{b} and thus represent a clinically relevant MHC-matched transplantation model.

The mice were divided into three cohorts: (1) a cohort that received B6 donor bone marrow cells at 5M and T cells at 2M only; (2) a cohort that received B6 donor bone marrow cells at 5M and T cells at 2M + subcutaneous administration of 100 mg/kg imipenem+cilastatin for three times per week from days 10-25 post-bone marrow transplantation; and (3) a cohort that received B6 donor bone marrow cells at 5M and T cells at 2M + subcutaneous administration of 100 mg/kg imipenem+cilastatin for three times per week from days 10-25 post-bone marrow transplantation + a liquid formulation of P2A (e.g., a beta-lactamase in a phosphate-buffered saline solution) via oral gavage at 50 mg/kg for three times per week from days 10-25 post-bone marrow transplantation.

**Figure 10** shows the results of the survival study with an endpoint of 40 days post-bone marrow transplantation. The data shows that P2A administration reduced antibiotic-exacerbated GVHD associated with bone marrow transplantation in terms of reducing mortality (p=0.0096, Mantel-Cox test).

### EQUIVALENTS

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth and as follows in the scope of the appended claims.

Those skilled in the art will recognize, or be able to ascertain, using no more than routine experimentation, numerous equivalents to the specific embodiments described specifically herein. Such equivalents are intended to be encompassed in the scope of the following claims.

### REFERENCES

The publications discussed herein are provided solely for their disclosure prior to the filing date of the present application. Nothing herein is to be construed as an admission that the present invention is not entitled to antedate such publication by virtue of prior invention.

As used herein, all headings are simply for organization and are not intended to limit the disclosure in any manner. The content of any individual section may be equally applicable to all sections.

## Claims

1. A beta-lactamase for use in reducing the incidence and/or severity of graft-versus-host disease (GVHD) in a subject in need thereof, optionally wherein the GVHD is acute, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase is the amino acid sequence of SEQ ID NO: 1, SEQ ID NO: 3, SEQ ID NO: 4, or SEQ ID NO: 6,
wherein the subject is being or has been administered an intravenous (IV) beta-lactam antibiotic, and
wherein (i) new colonization within the intestinal microbiome of the subject is prevented, (ii) expansion of colonization within the intestinal microbiome of the subject is prevented, and/or (iii) monodomination of the intestinal microbiome is prevented.

2. The beta-lactamase for use of claim 1, wherein (i) the subject is a transplant recipient, (ii) the subject is a recipient of allogeneic hematopoietic stem cell transplantation, (iii) the subject is a recipient of bone marrow cells, peripheral blood cells, or umbilical cord cells, and/or (iv) the beta-lactam antibiotic is selected from penicillins (e.g. piperacillin/tazobactam), cephalosporins (e.g. cefepime), and/or carbapenems (e.g. meropenem; imipenem/cilastatin).

3. The beta-lactamase for use of claim 2, wherein the beta-lactamase is administered prior to the transplant, and/or the beta-lactamase is administered subsequent to the transplant.

4. The beta-lactamase for use of any one of the above claims, wherein intestinal dysbiosis is reduced or eliminated in the subject.

5. The beta-lactamase for use of claim 4, wherein the microbiome colonization, expansion of colonization, or monodomination comprises one or more multi-drug resistant organisms, optionally wherein the one or more multi-drug resistant organisms is selected from *Aeromonas hydrophila, Bacillus,* e.g., *Bacillus cereus, Bifidobacterium, Bordetella, Borrelia, Brucella, Burkholderia, C. difficile, Campylobacter,* e.g., *Campylobacter fetus* and *Campylobacter jejuni, Chlamydia, Chlamydophila, Clostridium,* e.g., *Clostridium botulinum, Clostridioides difficile* (formerly *Clostridium difficile),* and *Clostridium perfringens, Corynebacterium, Coxiella, Ehrlichia, Enterobacteriaceae,* e.g., Carbapenem-resistant *Enterobacteriaceae* (CRE) and Extended Spectrum Beta-Lactamase producing *Enterobacteriaceae* (ESBL-E), fluoroquinolone-resistant *Enterobacteriaceae, Enterococcus,* e.g., vancomycin-resistant *enterococcus spp.,* extended spectrum beta-lactam resistant *Enterococci* (ESBL), and Vancomycin-resistant *Enterococci* (VRE), *Escherichia,* e.g., enteroaggregative *Escherichia coli,* enterohemorrhagic *Escherichia coli,* enteroinvasive *Escherichia coli,* enteropathogenic E. *coli,* enterotoxigenic *Escherichia coli* (such as but not limited to LT and/or ST), *Escherichia coli* 0157:1-17, and multi-drug resistant bacteria *Escherichia coli, Francisella, Haemophilus, Helicobacter,* e.g., *Helicobacter pylori, Klebsiella,* e.g., *Klebsiellia pneumonia* and multi-drug resistant bacteria *Klebsiella, Legionella, Leptospira, Listeria,* e.g., *Lysteria monocytogenes, Morganella, Mycobacterium, Mycoplasma, Neisseria, Orientia, Plesiomonas shigelloides,* Antibiotic-resistant *Proteobacteria, Proteus, Pseudomonas, Rickettsia, Salmonella,* e.g., *Salmonella paratyphi, Salmonella spp.,* and *Salmonella typhi, Shigella,* e.g., *Shigella spp., Staphylococcus,* e.g., *Staphylococcus aureus* and *Staphylococcus spp., Streptococcus, Treponema, Vibrio,* e.g., *Vibrio cholerae, Vibrio parahaemolyticus, Vibrio spp.,* and *Vibrio vulnificus,* and *Yersinia,* e.g., *Yersinia enterocolitica,* optionally wherein the microbiome colonization, expansion of colonization, or monodomination comprises overgrowth of enterococcal species, optionally wherein the microbiome colonization, expansion of colonization, or monodomination comprises vancomycin-resistant *enterococci* (VRE).

6. The beta-lactamase for use of any of claims 1-5, wherein the dose or length of dosing or dosing frequency of therapeutic agents to treat GVHD is reduced, optionally wherein the therapeutic agent to treat GVHD is a steroid, optionally methylprednisolone or prednisone.

7. The beta-lactamase for use of any one of the above claims, wherein (i) the beta-lactamase is released in the small intestine, optionally wherein the beta-lactamase is released at one or more of the duodenum, jejunum, ileum, and/or the ileocecal junction, or (ii) the beta-lactamase is released in the large intestine, optionally wherein the beta-lactamase is released at one or more of the cecum, ascending, transverse, descending or sigmoid portions of the colon, and rectum.

8. The beta-lactamase for use of any one of the above claims, wherein (i) the beta-lactamase is formulated with a modified-release coating that is substantially stable in gastric fluid, (ii) the beta-lactamase is formulated with a modified-release coating having a solubility that is pH-dependent, (iii) the beta-lactamase is formulated with a modified-release coating having a time-dependent erosion profile, (iv) the beta-lactamase is formulated with a modified-release coating that is degraded by a microbial enzyme present in the gut flora, and/or (v) the beta-lactamase is formulated as a capsule or a tablet, optionally wherein the beta-lactamase is formulated for oral administration.

9. A beta-lactamase for use in preventing or reducing the incidence of colonization, expansion of colonization, or monodomination and/or infection by one or more multi-drug resistant pathogens in a subject that is a transplant recipient, wherein the beta-lactamase has an amino acid sequence of at least 95% identity with SEQ ID NO: 1, SEQ ID NO: 3, SEQ ID NO: 4, or SEQ ID NO: 6, optionally wherein the beta-lactamase is the amino acid sequence of SEQ ID NO: 1, SEQ ID NO: 3, SEQ ID NO: 4, or SEQ ID NO: 6, and
wherein the subject is being or has been administered an intravenous (IV) beta-lactam antibiotic.

10. The beta-lactamase for use of claim 9, wherein (i) the subject has undergone prolonged hospitalization and/or has increased risk of death from the colonization and/or infection, (ii) the subject is a recipient of allogeneic hematopoietic stem cell transplantation, (iii) the subject is a recipient of bone marrow cells, peripheral blood cells, or umbilical cord cells, and/or (iv) the beta-lactam antibiotic is selected from penicillins (e.g. piperacillin/tazobactam), cephalosporins (e.g. cefepime), and/or carbapenems (e.g. meropenem; imipenem/cilastatin).

11. The beta-lactamase for use of claim 10, wherein the beta-lactamase is administered prior to the transplant, and/or the beta-lactamase is administered subsequent to the transplant.

12. The beta-lactamase for use of any one of claims 9-11, wherein (i) intestinal dysbiosis is reduced or eliminated in the subject, (ii) new colonization within the intestinal microbiome of the subject is prevented, (iii) expansion of colonization within the intestinal microbiome of the subject is prevented, and/or (iv) monodomination of the intestinal microbiome is prevented, optionally wherein the microbiome colonization, expansion of colonization, or monodomination comprises one or more multi-drug resistant organisms, optionally wherein the one or more multi-drug resistant organisms is selected from *Aeromonas hydrophila, Bacillus,* e.g., *Bacillus cereus, Bifidobacterium, Bordetella, Borrelia, Brucella, Burkholderia, C. difficile, Campylobacter,* e.g., *Campylobacter fetus* and *Campylobacter jejuni, Chlamydia, Chlamydophila, Clostridium,* e.g., *Clostridium botulinum, Clostridioides difficile* (formerly *Clostridium difficile),* and *Clostridium perfringens, Corynebacterium, Coxiella, Ehrlichia, Enterobacteriaceae,* e.g., Carbapenem-resistant *Enterobacteriaceae* (CRE) and Extended Spectrum Beta-Lactamase producing *Enterobacteriaceae* (ESBL-E), fluoroquinolone-resistant *Enterobacteriaceae, Enterococcus,* e.g., vancomycin-resistant *enterococcus spp.,* extended spectrum beta-lactam resistant *Enterococci* (ESBL), and Vancomycin-resistant *Enterococci* (VRE), *Escherichia,* e.g., enteroaggregative *Escherichia coli,* enterohemorrhagic *Escherichia coli,* enteroinvasive *Escherichia coli,* enteropathogenic E. *coli,* enterotoxigenic *Escherichia coli* (such as but not limited to LT and/or ST), *Escherichia coli* 0157:1-17, and multi-drug resistant bacteria *Escherichia coli, Francisella, Haemophilus, Helicobacter,* e.g., *Helicobacter pylori, Klebsiella,* e.g., *Klebsiellia pneumonia* and multi-drug resistant bacteria *Klebsiella, Legionella, Leptospira, Listeria,* e.g., *Lysteria monocytogenes, Morganella, Mycobacterium, Mycoplasma, Neisseria, Orientia, Plesiomonas shigelloides,* Antibiotic-resistant *Proteobacteria, Proteus, Pseudomonas, Rickettsia, Salmonella,* e.g., *Salmonella paratyphi, Salmonella spp.,* and *Salmonella typhi, Shigella,* e.g., *Shigella spp., Staphylococcus,* e.g., *Staphylococcus aureus* and *Staphylococcus spp., Streptococcus, Treponema, Vibrio,* e.g., *Vibrio cholerae, Vibrio parahaemolyticus, Vibrio spp.,* and *Vibrio vulnificus,* and *Yersinia,* e.g., *Yersinia enterocolitica.*

13. The beta-lactamase for use of claim 12, wherein (i) intestinal colonization by VRE is reduced, (ii) a bloodstream infection by VRE is reduced, (iii) the dose or length of dosing or dosing frequency of therapeutic agents to treat VRE colonization and/or infection is reduced, (iv) intestinal colonization by Carbapenem-resistant *Enterobacteriaceae* (CRE) is reduced, (v) a bloodstream infection by CRE is reduced, and/or (vi) the dose or length of dosing or dosing frequency of therapeutic agents to treat CRE colonization and/or infection is reduced.

14. The beta-lactamase for use of any one of the above claims, wherein (i) the beta-lactamase is released in the small intestine, optionally wherein the beta-lactamase is released at one or more of the duodenum, jejunum, ileum, and/or the ileocecal junction, and/or (ii) the beta-lactamase is released in the large intestine, optionally wherein the beta-lactamase is released at one or more of the cecum, ascending, transverse, descending or sigmoid portions of the colon, and rectum.

15. The beta-lactamase for use of any one of the above claims, wherein (i) the beta-lactamase is formulated with a modified-release coating that is substantially stable in gastric fluid, (ii) the beta-lactamase is formulated with a modified-release coating having a solubility that is pH-dependent, (iii) the beta-lactamase is formulated with a modified-release coating having a time-dependent erosion profile, (iv) the beta-lactamase is formulated with a modified-release coating that is degraded by a microbial enzyme present in the gut flora, and/or (v) the beta-lactamase is formulated as a capsule or a tablet, optionally wherein the beta-lactamase is formulated for oral administration.

## Patentansprüche

1. Beta-Lactamase zur Verwendung bei der Verringerung des Auftretens und/oder der Schwere einer Transplantat-gegen-Wirt-Krankheit (GVHD) bei einer Person, die diese benötigt, wobei die GVHD optional akut ist, wobei die Beta-Lactamase eine Aminosäuresequenz von mindestens 95 % Identität mit SEQ ID NO: 1, SEQ ID NO: 3, SEQ ID NO: 4 oder SEQ ID NO: 6 aufweist, wobei die Beta-Lactamase optional die Aminosäuresequenz von SEQ ID NO: 1, SEQ ID NO: 3, SEQ ID NO: 4 oder SEQ ID NO: 6 ist,
wobei der Person ein intravenöses (IV) Beta-Lactam-Antibiotikum verabreicht wird oder wurde, und
wobei (i) eine neue Besiedlung des Darmmikrobioms der Person verhindert wird, (ii) eine Ausbreitung der Besiedlung im Darmmikrobiom der Person verhindert wird, und/oder (iii) eine Monodomination des Darmmikrobioms verhindert wird.

2. Beta-Lactamase zur Verwendung nach Anspruch 1, wobei (i) die Person ein Transplantatempfänger ist, (ii) die Person ein Empfänger einer allogenen hämatopoetischen Stammzelltransplantation ist, (iii) die Person ein Empfänger von Knochenmarkzellen, peripheren Blutzellen oder Nabelschnurzellen ist, und/oder (iv) das Beta-Lactam-Antibiotikum ausgewählt ist aus Penicillinen (z. B. Piperacillin/Tazobactam), Cephalosporinen (z. B. Cefepime) und/oder Carbapenemen (z. B. Meropenem; Imipenem/Cilastatin).

3. Beta-Lactamase zur Verwendung nach Anspruch 2, wobei die Beta-Lactamase vor der Transplantation verabreicht wird und/oder die Beta-Lactamase nach der Transplantation verabreicht wird.

4. Beta-Lactamase zur Verwendung nach einem der vorstehenden Ansprüche, wobei die Darmdysbiose in der Person verringert oder beseitigt wird.

5. Beta-Lactamase zur Verwendung nach Anspruch 4, wobei die Mikrobiombesiedlung, die Ausbreitung der Besiedlung oder die Monodominanz einen oder mehrere multiresistente Organismen umfasst, wobei der eine oder die mehreren multiresistenten Organismen optional aus *Aeromonas hydrophila, Bacillus,* z. B. *Bacillus cereus, Bifidobacterium, Bordetella, Borrelia, Brucella, Burkholderia, C. difficile, Campylobacter,* z. B. *Campylobacter fetus* und *Campylobacter jejuni, Chlamydia, Chlamydophila, Clostridium,* z. B. *Clostridium botulinum, Clostridioides difficile* (früher *Clostridium difficile)* und *Clostridium perfringens, Corynebacterium, Coxiella, Ehrlichia, Enterobacteriaceae,* z. B. Carbapenem-resistente *Enterobacteriaceae* (CRE) und Beta-Lactamase produzierende *Enterobacteriaceae* (ESBL-E) mit erweitertem Spektrum, Fluorchinolon-resistente *Enterobacteriaceae, Enterococcus,* z. B. Vancomycin-resistente *Enterococcus spp.,* Beta-Lactam-resistente *Enterococci* (ESBL) mit erweitertem Spektrum und Vancomycin-resistente *Enterococci* (VRE), *Escherichia,* z. B. enteroaggregative *Escherichia coli,* enterohämorrhagische *Escherichia coli,* enteroinvasive *Escherichia coli,* enteropathogene E. *coli,* enterotoxigene *Escherichia coli* (beispielsweise, ohne darauf beschränkt zu sein, LT und/oder ST), *Escherichia coli* 0157:1-17 und multiresistente Bakterien *Escherichia coli, Francisella, Haemophilus, Helicobacter,* z. B. *Helicobacter pylori, Klebsiella,* z. B. *Klebsiellia pneumonia* und multiresistente Bakterien *Klebsiella, Legionella, Leptospira, Listeria,* z. B. *Lysteria monocytogenes, Morganella, Mycobacterium, Mycoplasma, Neisseria, Orientia, Plesiomonas shigelloides,* Antibiotika-resistente *Proteobakterien, Proteus, Pseudomonas, Rickettsia, Salmonella,* z. B. *Salmonella paratyphi, Salmonella spp.* und *Salmonella typhi, Shigella,* z.B. *Shigella spp., Staphylococcus,* z. B. *Staphylococcus aureus* und *Staphylococcus spp., Streptococcus, Treponema, Vibrio,* z. B. *Vibrio cholerae, Vibrio parahaemolyticus, Vibrio spp.* und *Vibrio vulnificus,* und *Yersinia,* z. B. *Yersinia enterocolitica,* wobei die Mikrobiombesiedlung, die Ausbreitung der Besiedlung oder die Monodominanz ein übermäßiges Wachstum von Enterokokken-Arten umfasst, wobei die Mikrobiombesiedlung, die Ausbreitung der Besiedlung oder die Monodominanz optional Vancomycin-resistente *Enterococci* (VRE) umfasst.

6. Beta-Lactamase zur Verwendung nach einem der Ansprüche 1-5, wobei die Dosis oder die Dauer der Verabreichung oder die Verabreichungshäufigkeit von therapeutischen Mitteln zur Behandlung von GVHD verringert wird, wobei es sich bei dem therapeutischen Mittel zur Behandlung von GVHD optional um ein Steroid, optional um Methylprednisolon oder Prednison, handelt.

7. Beta-Lactamase zur Verwendung nach einem der vorstehenden Ansprüche, wobei (i) die Beta-Lactamase im Dünndarm freigesetzt wird, wobei die Beta-Lactamase optional an einem oder mehreren von Duodenum, Jejunum, Ileum und/oder der Ileozökalkreuzung freigesetzt wird, oder (ii) die Beta-Laktamase im Dickdarm freigesetzt wird, wobei die Beta-Laktamase optional in einem oder mehreren vom Zökum, dem aufsteigenden, querverlaufenden, absteigenden oder sigmoiden Abschnitt des Dickdarms oder dem Rektum freigesetzt wird.

8. Beta-Lactamase zur Verwendung nach einem der vorstehenden Ansprüche, wobei (i) die Beta-Lactamase mit einer Beschichtung mit modifizierter Freisetzung formuliert ist, die in Magenflüssigkeit im Wesentlichen stabil ist, (ii) die Beta-Lactamase mit einer Beschichtung mit modifizierter Freisetzung formuliert ist, deren Löslichkeit pH-abhängig ist, (iii) die Beta-Lactamase mit einer Beschichtung mit modifizierter Freisetzung formuliert ist, die ein zeitabhängiges Erosionsprofil aufweist, (iv) die Beta-Lactamase mit einer Beschichtung mit modifizierter Freisetzung formuliert ist, die durch ein in der Darmflora vorhandenes mikrobielles Enzym abgebaut wird, und/oder (v) die Beta-Lactamase als Kapsel oder Tablette formuliert ist, wobei die Beta-Lactamase optional zur oralen Verabreichung formuliert ist.

9. Beta-Lactamase zur Verwendung bei der Verhinderung oder Verringerung des Auftretens der Besiedlung, Ausbreitung der Besiedlung oder Monodomination und/oder Infektion durch einen oder mehrere multiresistente Krankheitserreger in einer Person, die ein Transplantatempfänger ist, wobei die Beta-Lactamase eine Aminosäuresequenz von mindestens 95 % Identität mit SEQ ID NO: 1, SEQ ID NO: 3, SEQ ID NO: 4 oder SEQ ID NO: 6 aufweist, wobei die Beta-Lactamase optional die Aminosäuresequenz von SEQ ID NO: 1, SEQ ID NO: 3, SEQ ID NO: 4 oder SEQ ID NO: 6 ist, und
wobei der Person ein intravenöses (IV) Beta-Lactam-Antibiotikum verabreicht wird oder wurde.

10. Beta-Lactamase zur Verwendung nach Anspruch 9, wobei (i) die Person einen längeren Krankenhausaufenthalt hinter sich hat und/oder ein erhöhtes Risiko hat, an der Besiedlung und/oder Infektion zu sterben, (ii) die Person ein Empfänger einer allogenen hämatopoetischen Stammzelltransplantation ist, (iii) die Person ein Empfänger von Knochenmarkzellen, peripheren Blutzellen oder Nabelschnurzellen ist und/oder (iv) das Beta-Lactam-Antibiotikum ausgewählt ist aus Penicillinen (z. B. Piperacillin/Tazobactam), Cephalosporinen (z. B. Cefepime) und/oder Carbapenemen (z. B. Meropenem; Imipenem/Cilastatin).

11. Beta-Lactamase zur Verwendung nach Anspruch 10, wobei die Beta-Lactamase vor der Transplantation verabreicht wird und/oder die Beta-Lactamase nach der Transplantation verabreicht wird.

12. Beta-Lactamase zur Verwendung nach einem der Ansprüche 9-11, wobei (i) die Darmdysbiose in der Person verringert oder beseitigt wird, (ii) eine neue Besiedlung des Darmmikrobioms der Person verhindert wird, (iii) eine Ausbreitung der Besiedlung im Darmmikrobiom der Person verhindert wird, und/oder (iv) eine Monodomination des Darmmikrobioms verhindert wird, wobei die Mikrobiombesiedlung, die Ausbreitung der Besiedlung oder die Monodomination optional einen oder mehrere multiresistente Organismen umfasst, wobei der eine oder die mehreren multiresistenten Organismen ausgewählt sind aus *Aeromonas hydrophila, Bacillus,* z. B. *Bacillus cereus, Bifidobacterium, Bordetella, Borrelia, Brucella, Burkholderia, C. difficile, Campylobacter,* z. B. *Campylobacter fetus* und *Campylobacter jejuni, Chlamydia, Chlamydophila, Clostridium,* z. B. *Clostridium botulinum, Clostridioides difficile* (früher *Clostridium difficile)* und *Clostridium perfringens, Corynebacterium, Coxiella, Ehrlichia, Enterobacteriaceae,* z. B. Carbapenem-resistente *Enterobacteriaceae* (CRE) und Beta-Lactamase produzierende *Enterobacteriaceae* (ESBL-E) mit erweitertem Spektrum, fluorchinolonresistente *Enterobacteriaceae, Enterococcus,* z. B. Vancomycin-resistente *Enterococcus spp.,* Beta-Laktam-resistente *Enterococci* mit erweitertem Spektrum (ESBL) und Vancomycin-resistente *Enterococci* (VRE), *Escherichia,* z. B, enteroaggregative *Escherichia coli,* enterohämorrhagische *Escherichia coli,* enteroinvasive *Escherichia coli,* enteropathogene E. *coli,* enterotoxigene *Escherichia coli (wie* beispielsweise, ohne darauf beschränkt zu sein, LT und/oder ST), *Escherichia coli* 0157:1-17, und multiresistente Bakterien *Escherichia coli, Francisella, Haemophilus, Helicobacter,* z. B. *Helicobacter pylori, Klebsiella,* z. B. *Klebsiellia pneumonia* und multiresistente Bakterien *Klebsiella, Legionella, Leptospira, Listeria,* z. B. *Lysteria monocytogenes, Morganella, Mycobacterium, Mycoplasma, Neisseria, Orientia, Plesiomonas shigelloides,* Antibiotika-resistente *Proteobakterien, Proteus, Pseudomonas, Rickettsia, Salmonella,* z. B. *Salmonella paratyphi, Salmonella spp.* und *Salmonella typhi, Shigella,* z. B. *Shigella spp., Staphylococcus,* z. B. *Staphylococcus aureus* und *Staphylococcus spp, Streptococcus, Treponema, Vibrio,* z. B. *Vibrio cholerae, Vibrio parahaemolyticus, Vibrio spp.* und *Vibrio vulnificus,* und *Yersinia,* z. B. *Yersinia enterocolitica.*

13. Beta-Lactamase zur Verwendung nach Anspruch 12, wobei (i) die Darmbesiedlung durch VRE verringert wird, (ii) eine Infektion des Blutstroms durch VRE verringert wird, (iii) die Dosis oder die Dauer der Verabreichung oder die Verabreichungshäufigkeit von therapeutischen Mitteln zur Behandlung der VRE-Besiedlung und/oder -Infektion verringert wird, (iv) die Besiedlung des Darms mit Carbapenem-resistenten *Enterobacteriacae* (CRE) verringert wird, (v) eine Infektion des Blutstroms mit CRE verringert wird und/oder (vi) die Dosis oder die Dauer der Dosierung oder die Dosierungshäufigkeit von therapeutischen Mitteln zur Behandlung der CRE-Besiedlung und/oder -Infektion verringert wird.

14. Beta-Lactamase zur Verwendung nach einem der vorstehenden Ansprüche, wobei (i) die Beta-Lactamase im Dünndarm freigesetzt wird, wobei die Beta-Lactamase optional an einem oder mehreren von Duodenum, Jejunum, Ileum und/oder der Ileozökalkreuzung freigesetzt wird, und/oder (ii) die Beta-Lactamase im Dickdarm freigesetzt wird, wobei die Beta-Lactamase in einem oder mehreren vom Zökum, dem aufsteigenden, querverlaufenden, absteigenden oder sigmoiden Abschnitt des Dickdarms oder dem Rektum freigesetzt wird.

15. Beta-Lactamase zur Verwendung nach einem der vorstehenden Ansprüche, wobei (i) die Beta-Lactamase mit einer Beschichtung mit modifizierter Freisetzung formuliert ist, die in Magenflüssigkeit im Wesentlichen stabil ist, (ii) die Beta-Lactamase mit einer Beschichtung mit modifizierter Freisetzung formuliert ist, deren Löslichkeit pH-abhängig ist, (iii) die Beta-Lactamase mit einer Beschichtung mit modifizierter Freisetzung formuliert ist, die ein zeitabhängiges Erosionsprofil aufweist, (iv) die Beta-Lactamase mit einer Beschichtung mit modifizierter Freisetzung formuliert ist, die durch ein in der Darmflora vorhandenes mikrobielles Enzym abgebaut wird, und/oder (v) die Beta-Lactamase als Kapsel oder Tablette formuliert ist, wobei die Beta-Lactamase optional zur oralen Verabreichung formuliert ist.

## Revendications

1. Bêta-lactamase pour une utilisation dans la réduction de l'incidence et/ou de la gravité de la maladie du greffon contre l'hôte (GVH) chez un sujet en ayant besoin, facultativement dans laquelle la GVH est aiguë, la bêta-lactamase ayant une séquence d'acides aminés d'au moins 95 % d'identité avec la SEQ ID N° : 1, la SEQ ID N° : 3, la SEQ ID N° : 4 ou la SEQ ID N° : 6, facultativement la bêta-lactamase étant la séquence d'acides aminés de la SEQ ID N° : 1, de la SEQ ID N° : 3, de la SEQ ID N° : 4 ou de la SEQ ID N° : 6,
dans laquelle le sujet reçoit ou a reçu un antibiotique bêta-lactame par voie intraveineuse (IV), et
dans laquelle (i) une nouvelle colonisation au sein du microbiome intestinal du sujet est empêchée, (ii) l'expansion de la colonisation au sein du microbiome intestinal du sujet est empêchée, et/ou (iii) la monodominance du microbiome intestinal est empêchée.

2. Bêta-lactamase pour une utilisation selon la revendication 1, dans laquelle (i) le sujet est un receveur de greffe, (ii) le sujet est un receveur d'une greffe allogénique de cellules souches hématopoïétiques, (iii) le sujet est un receveur de cellules de moelle osseuse, de cellules de sang périphérique ou de cellules de cordon ombilical, et/ou (iv) l'antibiotique bêta-lactame est choisi parmi les pénicillines (par exemple, pipéracilline/tazobactam), les céphalosporines (par exemple, céfépime) et/ou les carbapénèmes (par exemple, méropénème ; imipénème/cilastatine).

3. Bêta-lactamase pour une utilisation selon la revendication 2, la bêta-lactamase étant administrée avant la transplantation, et/ou la bêta-lactamase étant administrée après la transplantation.

4. Bêta-lactamase pour une utilisation selon l'une quelconque des revendications précédentes, dans laquelle la dysbiose intestinale est réduite ou éliminée chez le sujet.

5. Bêta-lactamase pour une utilisation selon la revendication 4, dans laquelle la colonisation du microbiome, l'expansion de la colonisation ou la monodominance comprend un ou plusieurs organismes multirésistants, facultativement dans laquelle les un ou plusieurs organismes multirésistants sont choisis parmi *Aeromonas hydrophila, Bacillus,* par exemple *Bacillus cereus, Bifidobacterium, Bordetella, Borrelia, Brucella, Burkholderia, C. difficile, Campylobacter,* par exemple *Campylobacter fetus* et *Campylobacter jejuni, Chlamydia, Chlamydophila, Clostridium,* par exemple *Clostridium botulinum, Clostridioides difficile* (anciennement *Clostridium difficile),* et *Clostridium perfringens, Corynebacterium, Coxiella, Ehrlichia, Enterobacteriaceae,* par exemple les entérobactéries résistantes aux carbapénèmes (CRE) et les entérobactéries productrices de bêta-lactamases à spectre étendu (BLSE), les entérobactéries résistantes aux fluoroquinolones, les entérocoques, par exemple, les entérocoques *(Enterococcus spp)* résistants à la vancomycine, les entérocoques résistants aux bêta-lactamines à spectre étendu (BLSE) et les entérocoques résistants à la vancomycine (ERV), *Escherichia,* par exemple *Escherichia coli* entéro-aggrégatif, *Escherichia coli* entérohémorragique, *Escherichia coli* entéro-invasif, *E. coli* entéropathogène, *Escherichia coli* entérotoxigène (tel que, mais sans s'y limiter, LT et/ou ST), *Escherichia coli* 0157:1-17, et bactéries multirésistantes *Escherichia coli, Francisella, Haemophilus, Helicobacter,* par exemple *Helicobacter pylori, Klebsiella,* par exemple *Klebsiellia pneumonia* et bactéries *Klebsiella* multirésistantes, *Legionella, Leptospira, Listeria,* par exemple *Lysteria monocytogenes, Morganella, Mycobacterium, Mycoplasma, Neisseria, Orientia, Plesiomonas shigelloides, Proteobacteria* résistantes aux antibiotiques, *Proteus, Pseudomonas, Rickettsia, Salmonella,* par exemple *Salmonella paratyphi, Salmonella spp.* et *Salmonella typhi, Shigella,* par exemple *Shigella spp., Staphylococcus,* par exemple *Staphylococcus aureus* et *Staphylococcus spp., Streptococcus, Treponema, Vibrio,* par exemple *Vibrio cholerae, Vibrio parahaemolyticus, Vibrio spp.* et *Vibrio vulnificus,* et *Yersinia,* par exemple *Yersinia enterocolitica,* facultativement dans laquelle la colonisation du microbiome, l'expansion de la colonisation ou la monodominance comprend la prolifération d'espèces d'entérocoques, facultativement dans laquelle la colonisation du microbiome, l'expansion de la colonisation ou la monodominance comprend des entérocoques résistants à la vancomycine (ERV).

6. Bêta-lactamase pour une utilisation selon l'une quelconque des revendications 1 à 5, dans laquelle la dose ou la durée d'administration ou la fréquence d'administration d'agents thérapeutiques pour traiter la GVH est réduite, facultativement dans laquelle l'agent thérapeutique pour traiter la GVH est un stéroïde, facultativement la méthylprednisolone ou la prednisone.

7. Bêta-lactamase pour une utilisation selon l'une quelconque des revendications précédentes, (i) la bêta-lactamase étant libérée dans l'intestin grêle, facultativement la bêta-lactamase étant libérée au niveau d'un ou plusieurs parmi le duodénum, le jéjunum, l'iléon et/ou la jonction iléo-caecale, ou (ii) la bêta-lactamase étant libérée dans le gros intestin, facultativement la bêta-lactamase étant libérée au niveau d'un ou plusieurs parmi le cæcum, les parties ascendante, transversale, descendante ou sigmoïde du côlon, et le rectum.

8. Bêta-lactamase pour une utilisation selon l'une quelconque des revendications précédentes, (i) la bêta-lactamase étant formulée avec un enrobage à libération modifiée qui est sensiblement stable dans le suc gastrique, (ii) la bêta-lactamase étant formulée avec un enrobage à libération modifiée présentant une solubilité qui dépend du pH, (iii) la bêta-lactamase étant formulée avec un enrobage à libération modifiée présentant un profil d'érosion qui dépend du temps, (iv) la bêta-lactamase étant formulée avec un enrobage à libération modifiée qui est dégradé par une enzyme microbienne présente dans la flore intestinale, et/ou (v) la bêta-lactamase étant formulée sous forme de capsule ou de comprimé, facultativement la bêta-lactamase étant formulée pour une administration par voie orale.

9. Bêta-lactamase pour une utilisation dans la prévention ou la réduction de l'incidence de la colonisation, de l'expansion de la colonisation ou de la monodominance et/ou de l'infection par un ou plusieurs agents pathogènes multirésistants chez un sujet receveur d'une transplantation, la bêta-lactamase ayant une séquence d'acides aminés présentant une identité d'au moins 95 % avec la SEQ ID N° : 1, la SEQ ID N° : 3, la SEQ ID N° : 4 ou la SEQ ID N° : 6, facultativement la bêta-lactamase étant la séquence d'acides aminés de la SEQ ID N° : 1, de la SEQ ID N° : 3, de la SEQ ID N° : 4 ou de la SEQ ID N° : 6, et
dans laquelle le sujet reçoit ou a reçu un antibiotique bêta-lactame par voie intraveineuse (IV).

10. Bêta-lactamase pour une utilisation selon la revendication 9, dans laquelle (i) le sujet a subi une hospitalisation prolongée et/ou présente un risque accru de décès dû à la colonisation et/ou à l'infection, (ii) le sujet est un receveur d'une greffe allogénique de cellules souches hématopoïétiques, (iii) le sujet est un receveur de cellules de moelle osseuse, de cellules de sang périphérique ou de cellules de cordon ombilical, et/ou (iv) l'antibiotique bêta-lactame est choisi parmi les pénicillines (par exemple, pipéracilline/tazobactam), les céphalosporines (par exemple, céfépime) et/ou les carbapénèmes (par exemple, méropénème ; imipénème/cilastatine).

11. Bêta-lactamase pour une utilisation selon la revendication 10, la bêta-lactamase étant administrée avant la transplantation, et/ou la bêta-lactamase étant administrée après la transplantation.

12. Bêta-lactamase pour une utilisation selon l'une quelconque des revendications 9 à **11,** dans laquelle (i) la dysbiose intestinale est réduite ou éliminée chez le sujet, (ii) la nouvelle colonisation au sein du microbiome intestinal du sujet est empêchée, (iii) l'expansion de la colonisation au sein du microbiome intestinal du sujet est empêchée, et/ou (iv) la monodominance du microbiome intestinal est empêchée, facultativement dans laquelle la colonisation du microbiome, l'expansion de la colonisation ou la monodominance comprend un ou plusieurs organismes multirésistants, facultativement dans laquelle les un ou plusieurs organismes multirésistants sont choisis parmi *Aeromonas hydrophila, Bacillus,* par exemple *Bacillus cereus, Bifidobacterium, Bordetella, Borrelia, Brucella, Burkholderia, C. difficile, Campylobacter,* par exemple *Campylobacter fetus* et *Campylobacter jejuni, Chlamydia, Chlamydophila, Clostridium,* par exemple *Clostridium botulinum, Clostridioides difficile* (anciennement *Clostridium difficile),* et *Clostridium perfringens, Corynebacterium, Coxiella, Ehrlichia, Enterobacteriaceae,* par exemple les entérobactéries résistantes aux carbapénèmes (CRE) et les entérobactéries productrices de bêta-lactamases à spectre étendu (BLSE), les entérobactéries résistantes aux fluoroquinolones, les entérocoques, par exemple, les entérocoques *(Enterococcus spp)* résistants à la vancomycine, les entérocoques résistants aux bêta-lactamines à spectre étendu (BLSE) et les entérocoques résistants à la vancomycine *(ERV), Escherichia,* par exemple *Escherichia coli* entéro-aggrégatif, *Escherichia coli* entérohémorragique, *Escherichia coli* entéro-invasif, *E. coli* entéropathogène, *Escherichia coli* entérotoxigène (tel que, mais sans s'y limiter, LT et/ou ST), *Escherichia coli* 0157:1-17, et bactéries multirésistantes *Escherichia coli, Francisella, Haemophilus, Helicobacter,* par exemple *Helicobacter pylori, Klebsiella,* par exemple *Klebsiellia pneumonia* et bactéries *Klebsiella* multirésistantes, *Legionella, Leptospira, Listeria,* par exemple *Lysteria monocytogenes, Morganella, Mycobacterium, Mycoplasma, Neisseria, Orientia, Plesiomonas shigelloides, Proteobacteria* résistantes aux antibiotiques, *Proteus, Pseudomonas, Rickettsia, Salmonella,* par exemple *Salmonella paratyphi, Salmonella spp.* et *Salmonella typhi, Shigella,* par exemple *Shigella spp., Staphylococcus,* par exemple *Staphylococcus aureus* et *Staphylococcus spp., Streptococcus, Treponema, Vibrio,* par exemple *Vibrio cholerae, Vibrio parahaemolyticus, Vibrio spp.* et *Vibrio vulnificus,* et *Yersinia,* par exemple *Yersinia enterocolitica.*

13. Bêta-lactamase pour une utilisation selon la revendication 12, dans laquelle (i) la colonisation intestinale par les ERV est réduite, (ii) une infection de la circulation sanguine par les ERV est réduite, (iii) la dose ou la durée d'administration ou la fréquence d'administration d'agents thérapeutiques pour traiter la colonisation et/ou l'infection par les ERV est réduite, (iv) la colonisation intestinale par les entérobactéries résistantes aux carbapénèmes (ERC) est réduite, (v) une infection de la circulation sanguine par des entérobactéries résistantes aux carbapénèmes (ERC) est réduite, et/ou (vi) la dose ou la durée d'administration ou la fréquence d'administration des agents thérapeutiques pour traiter la colonisation et/ou l'infection par des ERC est réduite.

14. Bêta-lactamase pour une utilisation selon l'une quelconque des revendications précédentes, (i) la bêta-lactamase étant libérée dans l'intestin grêle, facultativement la bêta-lactamase étant libérée au niveau d'un ou plusieurs parmi le duodénum, le jéjunum, l'iléon et/ou la jonction iléo-caecale, et/ou (ii) la bêta-lactamase étant libérée dans le gros intestin, facultativement la bêta-lactamase étant libérée au niveau d'un ou plusieurs parmi le cæcum, les parties ascendante, transversale, descendante ou sigmoïde du côlon, et le rectum.

15. Bêta-lactamase pour une utilisation selon l'une quelconque des revendications précédentes, (i) la bêta-lactamase étant formulée avec un enrobage à libération modifiée qui est sensiblement stable dans le suc gastrique, (ii) la bêta-lactamase étant formulée avec un enrobage à libération modifiée présentant une solubilité qui dépend du pH, (iii) la bêta-lactamase étant formulée avec un enrobage à libération modifiée présentant un profil d'érosion qui dépend du temps, (iv) la bêta-lactamase étant formulée avec un enrobage à libération modifiée qui est dégradé par une enzyme microbienne présente dans la flore intestinale, et/ou (v) la bêta-lactamase étant formulée sous forme de capsule ou de comprimé, facultativement la bêta-lactamase étant formulée pour une administration par voie orale.
